# EUROPEAN PATENT SPECIFICATION

(11) **EP 2 026 851 B1**
(45) Date of publication and mention of the grant of the patent: **08.04.2015**
(21) Application number: 06736649.2
(22) Date of filing: 27.02.2006
(51) Int. Cl.: A61L 15/46, A61L 15/18, A61K 33/08, A01N 25/34, A01N 59/16, D06M 11/42

(54) **WOUND CARE DEVICE**
WUNDPFLEGEVORRICHTUNG
DISPOSITIF DE SOINS DE PLAIES

(30) Priority: 28.02.2005 US 68639; 03.02.2006 US 347522
(43) Date of publication of application: 25.02.2009
(60) Divisional application: 13153258.2
(73) Proprietor: Milliken & Company, Spartanburg, SC 29303 (US)
(72) Inventor: CANADA, T. Andrew, Campobello, SC 29322 (US); STURM, Raymond C., Spartanburg, SC 29301 (US); WIENCEK, K. Mark, Inman, SC 29349 (US); KREIDER, Jason L., Boiling Springs, SC 29316 (US); SCHUETTE, Robert L., Duncan, SC 29334 (US); COWAN, Martin E., Moore, SC 29369 (US)
(74) Representative: Hoffmann Eitle
(86) International application number: PCT/US2006/007368
(87) International publication number: WO 2006/094098

(56) References cited:
- EP-A- 0 242 415
- WO-A-2005/018543
- WO-A-2006/036581
- DD-A1- 155 873
- GB-A- 2 321 216
- US-A1- 2005 037 057
- US-A1- 2005 037 680
- US-B2- 6 584 668

## Description

### TECHNICAL FIELD

This disclosure relates to wound care devices, which may have a topically applied silver-based antimicrobial finish. More specifically, this disclosure relates to topical antimicrobial finishes with silver ion-releasing mechanisms and to articles having these antimicrobial finishes. The application of the present finish to a substrate results in an antimicrobial product that substantially retains its initial color. This highly desirable feature contrasts sharply with products that are commercially available today and that may be described in the prior art, which are silver-based antimicrobial articles that typically appear as dark colored substrates.

The present finish may be applied to a target substrate to provide a single layer antimicrobial wound care device. Alternatively, a silver-containing layer, as will be described herein, may be combined with one or more additional layers to provide a composite antimicrobial wound care device.

A silver-based antimicrobial finish is topically applied to a fabric comprised of fibers. Such fibrous substrates provide sufficient surface area onto which the silver ion-releasing antimicrobial may adhere, thus making available an amount of surface-available silver on the wound care device that is sufficient for promotion of wound healing.

A wound care device is disclosed which contains one-way capillary force "pumps" that are capable of transporting fluid, such as wound exudate, away from a wound site to the opposite side of the wound care device, which functions as a segregated fluid reservoir. This fluid transport mechanism generally aids in reducing wound maceration by removing excess wound fluid and, consequently, the protease enzymes and infectious bacteria contained within the wound fluid. The wound care device performs this function, often for multiple consecutive days, without a loss in the physical integrity of the wound care device. In addition to providing a uni-directional fluid transport mechanism, the wound care device may contain a topically applied silver-based antimicrobial finish which provides certain levels of antimicrobial agent to the wound in order to reduce infection from the wound site and control microbial growth in the wound care dressing. Exemplary topical antimicrobial finishes include silver ion-releasing compounds.

The wound care device may be comprised of a knit construction characterized in that polyester fiber is primarily present on the wound contact surface and nylon fiber is primarily present on the fluid reservoir surface. A third fiber, such as an elastomeric polyurethane known by the tradename LYCRA®, may also be included in order to provide some amount of elasticity to the wound care device. The wound care device provides a uni-directional flow of fluid away from the wound and into the nylon fluid reservoir. Since the fluid is partitioned away from the wound, the propensity of localized wound maceration is reduced. Furthermore, due to the construction of the wound care device, at least in part, the device maintains a high degree of physical integrity when saturated with fluid.

In whatever form (that is, single layer or composite) and being made from whatever materials (natural or synthetic), the present wound care device substrate is unique in that it substantially retains its original color through processing, irradiation, and storage, compared to similar articles produced from other silver-based compounds present at the levels required for effective wound treatment. Further, the ability to create essentially white-colored, silver-based antimicrobial textiles, which are capable of moisture management, affords the opportunity to provide silver-based antimicrobial substrates in a wide variety of light colors previously unavailable.

### BACKGROUND

Silver-containing antimicrobials have been incorporated into wound care devices and are rapidly gaining acceptance in the medical industry as a safe and effective means of controlling microbial growth in the wound bed, often resulting in improved healing. It is known that placing surface-available silver in contact with a wound allows the silver to enter the wound and become absorbed by undesirable bacteria and fungi that grow and prosper in the warm, moist environment of the wound site. Once absorbed, the silver ions kill microbes, resulting in treatment of infected wounds or the prevention of infection in at-risk wounds.

However, existing wound care devices with active silver components typically have two major shortcomings. First, the presence of silver tends to cause discoloration of the substrate into which the silver is incorporated. A second problem with existing devices is that their construction fails to provide adequate movement, or transport, of moisture (such as wound exudate) away from the wound site. The device of the present disclosure addresses and overcomes these deficiencies.

Discoloration of substrates containing silver ion-releasing compounds is known to occur. Silver ion-releasing compounds, such as those selected from the group consisting of silver ion exchange materials (e.g. zirconium phosphates and zeolites), silver particles (e.g. silver metal, nanosilver, colloidal silver), silver salts (e.g. AgCl, Ag₂CO₃), silver glass, and mixtures thereof, are generally susceptible to discoloration and have a tendency to alter the color of the substrate in which they are incorporated. More specifically, excess silver ions can combine with available anions to form colored, precipitated salts. Many of these silver salts darken upon exposure to light as a result of the photo-reduction of silver ion to silver metal. When such compounds are incorporated into prior wound care devices at levels required to deliver effective performance, the color of the substrate is darkened as a result of the presence of the silver compounds.

This dark color of the substrate is especially problematic in the medical industry, and specifically in wound care devices, where examination of the wound site (as well as the bandage or dressing covering the wound) can provide important indicators of the effectiveness of the treatment administered to a particular wound. As such, evidence of color on the wound care device may indicate infection at a wound site (e.g., purulent green discharge being indicative of infection with a *Pseudomonas* species of bacteria), uncontrolled bleeding (e.g., red discharge), or debrided eschar (e.g., brown slough), with such discoloration being more readily apparent in a white, or similarly light-colored, wound care device.

However, if the device has a dark color as manufactured due to a high loading of silver antimicrobial contained within or on the wound care device itself, the relevant color (e.g., from blood or infected exudates) during use may be more difficult for the caregiver to assess. Thus, it is important to those in the medical industry that the wound care device itself does not become discolored merely because silver ions are undergoing photo-reduction, as such discoloration could lead to confusion as to the effectiveness of the treatment being administered to the wound. Accordingly, a stable silver-containing antimicrobial finish on a wound care device, which substantially maintains the original color of the device, is most desirable.

There have been various attempts by others to create silver ion-releasing wound care devices. In many wound care devices, the silver antimicrobial is present throughout the entire cross section of the device. For example, silver antimicrobials have been adapted for incorporation within melt-spun synthetic fibers in order to provide certain fabrics that selectively and inherently exhibit antimicrobial characteristics. Commercial examples include DAK's antimicrobial polyester fiber under the tradename STERIPURE® and Unifi's antimicrobial nylon fiber under the tradename A.M.Y. In another example, silver antimicrobials have been adapted for incorporation within bi-component, core/sheath fibers as taught within US Patent Nos. 6,723,428 and 6,841,244.

However, the melt-spun fibers described above are expensive to produce due to the large amount of silver-based compound required to provide sufficient antimicrobial activity, especially in light of the relative lack of migration of the compound from within the fiber itself to its surface. As such, when these silver-containing fibers are combined to form a wound care device, the silver located on the interior of the fiber may never reach the wound site during the useful life of the device to provide any advantage to the healing process. Thus, this approach results in an inefficient and expensive use of silver in wound care devices, and it is even likely that the amount of silver released from the fibers is inadequate for promoting the healing process.

Others have attempted to provide composite, multi-layered wound care devices. An example of this approach is marketed by Smith and Nephew under the tradename ACTICOAT^{®}. This wound care device is comprised of three layers-a layer of polyethylene film, a middle layer of rayon/polyester blend nonwoven fabric, and a second layer of film. Nano-crystalline silver particles are deposited onto the film layers to provide an antimicrobial wound care device. However, this technology generally fails to impart desirable release of silver from the device, while the device itself exhibits a metallic blue coloration. This product has the potential to initially release large amounts of silver from the wound care device, often in the form of silver flakes, which may enter the wound bed and may lead to irritation of the wound.

Another product available to consumers, provided by Johnson & Johnson under the trademark ACTISORB®, is a highly porous, silver-impregnated charcoal cloth, sandwiched between two nylon nonwoven layers. This product generally provides very low release of silver, and the device itself is black initially due to the presence of a silver charcoal active ingredient.

Yet another example is manufactured by Argentum under the trademark SILVERLON®. Silver, via a solution of silver nitrate, is reduced and deposited on sensitized polymeric fibers (typically nylon). The silver-laden polyamide is then attached to a subsequent fiber layer. Because of the nature of this technology, it is difficult to control the amount of silver deposited on the substrate, causing this product to show dark coloration as well.

In all cases where the wound care device is colored (e.g., metallic blue, brown, gray, black) by the addition of silver to the device, a situation exists in which medical personnel and/or the users thereof will have greater difficulty in caring for wound sites and monitoring the healing process. To this point, attempts to create a fiber-based wound care device having both effective antimicrobial properties and its original color (preferably white) have been unsuccessful.

Because treatments in which the silver antimicrobial is incorporated into the fiber have been found ineffective, another approach was necessary. A topical finish for textile substrates, such as a fabric, is desirable because it permits treatment of a fabric's individual fibers before or after weaving, knitting, and the like, in order to provide greater functionality to the target yarn and enhanced likelihood of effectiveness in a wound care device. Such a finish should be capable of releasing a desired amount of silver to the wound from a substrate whose color has not been substantially altered by the addition of a silver antimicrobial. Furthermore, it is desirable in the case of metallic silver that a metallized treatment be electrically non-conductive on the target fabric, fiber, or yarn.

Methods of topically applying a silver-based antimicrobial finish to textile substrates are described in commonly assigned U.S. Patent Nos. 6,584,668; 6,821,936; and 6,946,433 and in commonly assigned U.S. Patent Application Serial Nos. 09/586,081; 09/589,179; 10/307,027, and 10/306,968. Details of many of these processes will be discussed below.

The present disclosure addresses and overcomes the problems of discoloration associated with silver-containing wound care devices described above. Whereas, historically, a silver antimicrobial has been incorporated into a melt or polymer matrix prior to the formation of a fiber or substrate, or silver metal is deposited on the surface to create a dark-colored antimicrobial substrate useful for wound care devices, the present disclosure describes a method for achieving an effective wound care device having a silver-based antimicrobial finish, which is topically applied to a target substrate without substantially altering the color of the device. The resultant wound care device provides desired release of silver to the wound site and, because of its unchanged color, offers benefits in terms of wound monitoring and manufacturing flexibility.

Thus, a silver-releasing wound care device having an L* value of at least about 68.0 (indicative of fabric lightness, as will be discussed herein) is useful in monitoring the treatment of a wound site, where the use of the device includes the steps of providing the present wound care device, applying the wound care device to the wound site, and monitoring the various stages of healing at the wound site (for example, as evidenced by changes in the type and amount of wound fluid on the wound care device).

In the medical field, and in the area of wound care particularly, it is well-established that many factors, including the amount of moisture present at a wound site, affects how quickly a wound will heal. Generally speaking, having an excessive amount of moisture present at a wound site, especially when combined with the warm environment provided by the body, leads to undesirable bacteria growth and production of protease enzymes in the wound. Such growth can cause further damage to healthy cells and delay the healing process. However, insufficient moisture at the wound site can cause eschar (scab) formation and scarring and may cause the wound care device, or medical dressing, to adhere to the wound. If the dressing adheres to the wound, subsequent removal of the dressing may cause undue discomfort to the patient as well as disrupt newly granulated tissue. Infection of the wound may also be compounded when a medical dressing is removed and portions of the dressing remain behind in the wound itself, particularly if the dressing is already colonized with pathogenic microbes. Thus, it is important that the dressing maintains its physical integrity when exposed to stress, such as during removal from the wound, in order to prevent additional complications and delays in healing.

Absorptive materials such as gauzes, hydrogels, swellable fibers, foams, woven textiles and the like have been incorporated into wound care devices for the purpose of controlling the wound moisture content. Fluids are generally absorbed by these types of materials by reversible capillary action or osmosis rather than by a one-way directional flow created by an inventive two-sided wound care device.

For example, US Patent No. 5,009,652 to Morgan et al. discloses a disposable laminated medical sponge that contains a thin film which is impervious to fluids and infectious agents. The medical sponge is designed to prevent the seepage of bodily fluids from one side of the sponge to the opposite side, since such seepage provides risk of infection for health-care workers having direct contact with patients.

US Patent No. 6,194,332 to Rock et al. discloses an antimicrobial composite fabric having a first inner fabric layer and a second outer fabric layer. The inner fabric layer may be comprised of polyester, acrylic or nylon fiber which has been rendered hydrophilic, such as by mechanical or chemical treatment. The hydrophilic inner fabric layer enables the transport of sweat from the inner fabric layer to the outer fabric layer. The fibers in the outer layer of the fabric may be blended with antimicrobial fibers in order to reduce the proliferation of bacteria in this layer. The fabric may be formed into a garment which provides reduced body odor. US Patent No. 6,602,811 to Rock et al. discloses a similar antimicrobial composite fabric, except that the second outer fabric layer also may be treated with an antimicrobial paste.

US Patent Application Publication No. 2004/0001880 to Bowler et al. discloses the use of gel forming fibers such as sodium carboxymethycellulose which can be incorporated into wound dressings. Silver ions may be incorporated into the fibers by combining them in a solution with a solvent prior to fiber formation. The dressing may be used as part of a larger dressing or a layer in a multi-layered dressing and need not be in direct contact with the wound.

The wound care device of the present invention takes advantage of a unique textile fabric construction which effectively isolates fluid away from the wound, which often results in improved healing. The differentiation that exists in a wound care device having a hydrophobic fiber on the wound contact side of the device and hydrophilic fiber on the fluid reservoir side of the device creates a unique one-way, directional flow of fluid and contaminants away from the wound.

The present disclosure addresses and overcomes the problems associated with moisture management described above. Whereas, historically, a gauze or foam medical dressing has been applied to a wound with at least some intent on absorbing fluids, the present disclosure describes a wound care device capable of creating a one-way, directional flow of fluid and contaminants away from the wound, without detrimentally causing excessive dryness of the wound and substantial adherence of the device to the wound. The wound care device additionally provides desired release of silver to the wound site for antimicrobial efficacy and, because of its unique construction, maintains its physical integrity when exposed to stress during ordinary use of the wound care device.

Thus, a wound care device for managing the movement of wound fluid from a wound site to the wound care device, where the wound care device has a wound contact surface and a wound fluid reservoir surface opposite the wound contact surface, is useful in monitoring the treatment of a wound site, where the use of the device includes the steps of providing the present wound care device, applying the wound care device to the wound site such that the wound contact surface is contiguous with the wound site, and monitoring the movement of wound fluid by uni-directionally transporting the wound fluid through the wound contact surface of the device to the wound fluid reservoir surface of the device. A finish containing a silver ion-releasing compound is further added to the wound care device.

The present wound care device optionally includes additional layers that may assist in boosting absorption capacity, such as, for example, one or more layers of cotton gauze, foam, alginate, carboxymethyl cellulose, and the like. These additional layers may or may not contain an antimicrobial agent.

For these reasons and others that will be described herein, the present effective silver wound care device represents a useful advance over the prior art.

### BRIEF DESCRIPTION OF THE DRAWINGS

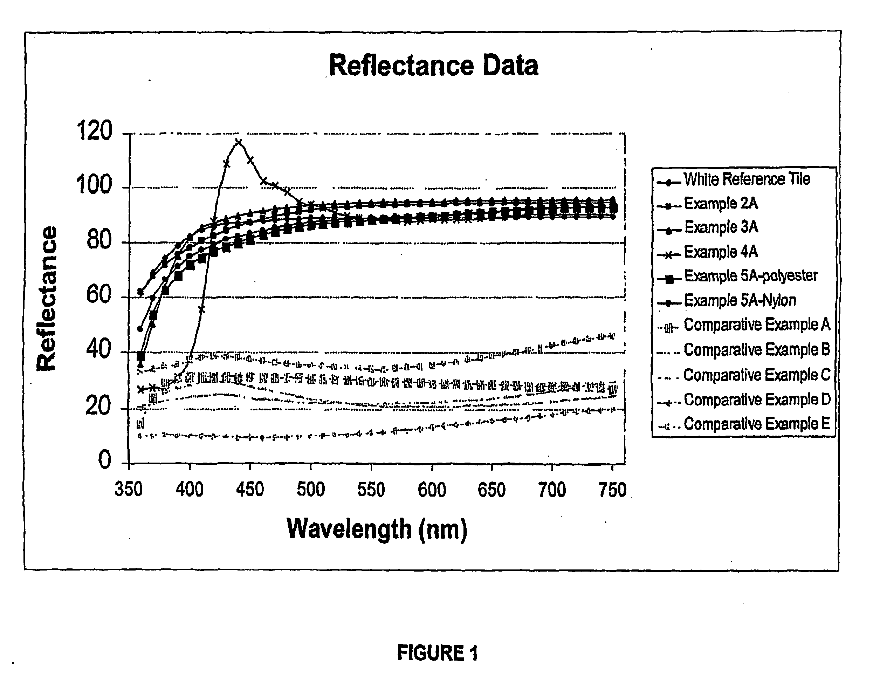

FIGURE 1 is a line graph depicting the reflectance of Examples 2A, 3A, 4A, 5B, and Comparative Examples A-E.

### DETAILED DESCRIPTION

The wound care device of the present invention is generally intended to be used for treatment of various wounds including, without limitation, partial thickness bums, incisions, skin grafts, donor sites, lacerations, abrasions, Stage I-IV pressure ulcers, vascular venous stasis, and diabetic ulcers.

### Definiflons and Terms

"Hydrophilic" is defined as having a strong affinity for or the ability to absorb water.

"Hydrophobic" is defined as lacking affinity for or the ability to absorb water.

"Non-electrically conductive" is defined as having a resistance in ohms per square inch of fabric of greater than about 10,000 ohms, preferably greater than about 100,000 ohms and most preferably greater than about 1 x 10⁹ ohms, when measured in accordance with AATCC Test Method 76-1978.

### Wound Care Substrates

Suitable substrates for receiving a topically applied silver-based antimicrobial finish include, without limitation, fibers, fabrics, and alginates. The fabric may be formed from fibers such as synthetic fibers, natural fibers, or combinations thereof. Synthetic fibers include, for example, polyester, acrylic, polyamide, polyolefin, polyaramid, polyurethane, regenerated cellulose (i.e., rayon), and blends thereof.

The term "polyamide" is intended to describe any long-chain polymer having recurring amide groups (-NH-CO-) as an integral part of the polymer chain. Examples of polyamides include nylon 6; nylon 6, 6; nylon 1, 1; and nylon 6, 10.

The term "polyester" is intended to describe any long-chain polymer having recurring ester groups (-C(O)-O-). Examples of polyesters include aromatic polyesters, such as polyethylene terephthalate (PET), polybutylene terephthalate (PBT), polytrimethylene terephthalate (PTT), and polytriphenylene terephthalate, and aliphatic polyesters, such as polylactic acid (PLA).

"Polyolefin" includes, for example, polypropylene, polyethylene, and combinations thereof. "Polyaramid" includes, for example, poly-*p*-phenyleneteraphthalamid (i.e., KEVLAR®), poly-*m*-phenyleneteraphthalamid (i.e., NOMEX®), and combinations thereof. Natural fibers include, for example, wool, cotton, flax, and blends thereof.

The fabric may be formed from fibers or yarns of any size, including microdenier fibers and yarns (fibers or yarns having less than one denier per filament). The fibers or yarns may have deniers that range from less than about 1 denier per filament to about 2000 denier per filament or more preferably, from less than about 1 denier per filament to about 500 denier per filament, or even more preferably, from less than about 1 denier per filament to about 300 denier per filament.

Furthermore, the fabric may be partially or wholly comprised of multi-component or bi-component fibers or yarns, which may be splittable, or which have been partially or fully split, along their length by chemical or mechanical action. The fabric may be comprised of fibers such as staple fiber, filament fiber, spun fiber, or combinations thereof.

The fabric may be of any variety, including but not limited to, woven fabric, knitted fabric, nonwoven fabric, or combinations thereof. The unique and interesting achievement realized through the present topical application is that the wound care device substantially maintains its original color, despite the presence of effective amounts of a silver-based antimicrobial agent. The elimination of color normally associated with the inclusion of silver-based antimicrobials is highly beneficial and desirable. The wound care devices (preferably, white-colored), as will be described herein, allow users thereof and their health care providers to monitor the exudates from the wound. Further, the present wound care devices exhibit long-term color stability (that is, their color does not change significantly over time while in production, transit, or storage). Finally, because the present wound care device is not discolored by the addition of the silver-based antimicrobial agent, a variety of substrate colors may be utilized or the finished wound care devices may be dyed or colored to any desired shade or hue with any type of colorant, such as, for example, pigments, dyes, tints, and the like.

For instance, the fabric used for the present wound care device may optionally be colored by a variety of dyeing techniques, such as high temperature jet dyeing with disperse dyes, vat dyeing, thermosol dyeing, pad dyeing, flexographic printing, transfer printing, screen printing, or any other technique that is common in the art for comparable textile products. If yarns or fibers are treated by the process of the current invention, they may be dyed by suitable methods prior to fabric formation, such as, for instance, by package dyeing or solution dyeing, or after fabric formation as described above, or they may be left undyed.

Other additives may be present on and/or within the target fabric or fiber, including antistatic agents, optical brightening compounds, opacifiers (such as titanium dioxide), nucleating agents, antioxidants, UV stabilizers, fillers, permanent press finishes, softeners, lubricants, curing accelerators, adhesives, odor control agents, molecular scavengers (such as compounds to bind endotoxins), and the like. The present fabrics may also be coated or printed or otherwise aesthetically modified in addition to being treated with the present antimicrobial compositions.

However, it should be noted that additives such as optical brightening compounds, opacifiers, dyes, and pigments are not required on or within the target substrate in order to achieve a white wound care device. These specific additives may be included merely for the purpose of enhancing the whiteness already exhibited by the wound care device.

Alginates from commercial sources are an alternative substrate, which may be used in place of fabrics. A typical process of manufacturing alginates involves crushing and washing of the raw material (i.e., seaweed) and dissolution of the extracted sodium alginate in water. A viscous solution is obtained that is extruded into a calcium chloride bath. Here, the sodium ions are replaced by calcium ions, and an insoluble calcium alginate is precipitated. Rinsing and dehydration then leads to the production of a fiber. Fibers may be formed from alginate by extruding or spinning the alginate from an aqueous solution. The fibers are then typically laid down in a web mat that can be incorporated into a wound care device.

An alginate web containing the calcium alginate fibers is placed on the wound in a dry state and begins to absorb the exudates. At this time, reverse ion exchange takes place, in which the calcium ions that are present in the alginate are gradually exchanged for sodium ions that are present in the blood and wound exudates. The fibers absorb large amounts of secretions, start to swell, and, in the presence of sodium ions, turn into a moist gel that fills and securely covers the wound.

In one embodiment of the invention, a commercially available nonwoven fabric is used to form the wound care device. Nonwovens are known in the textile industry as an alternative to traditional woven or knit fabrics. To create a nonwoven fabric, a filament web must be created and then consolidated. In one method, staple fibers are formed into a web through the carding process, which can occur in either wet or dry conditions. Alternatively, continuous filaments, which are formed by extrusion, may be used in the formation of the web. The web is then consolidated, and/or bonded, by means of needle-punching, thermal bonding, chemical bonding, or hydroentangling. A second consolidation method may also be employed such as thermal bonding.

One potentially preferred substrate for use in the wound care device of the present disclosure is a nonwoven fabric formed of continuous splittable filaments that are extruded as a web and then consolidated. This nonwoven fabric is described in U.S. Patent Nos. 5,899,785 and 5,970,583, both assigned to Firma Carl Freudenberg of Weinheim, Germany. Preferably, the nonwoven web is consolidated through hydroentanglement, and, more preferably, through hydroentanglement followed by thermal, or point, bonding. The continuous composite filaments are obtained by means of a controlled spinning process, and the hydroentanglement process mechanically splits at least some, if not most, of the composite filaments into their elementary components. This structure of split fibers provides a greater surface area onto which the present silver-based antimicrobial compound may be applied and, therefore, greater amounts of surface-available silver that may contact the wound.

A knit pattern that is suitable for making the fabric that comprises the wound care device of the present invention is a jersey knit. A jersey knit is a circular or flat-knit fabric made with a plain stitch in which the loops intermesh in only one direction. As a result, the appearance of the face and the back of the jersey fabric is wholly different. Thus, by utilizing a jersey knit to form a fabric comprised of polyester, nylon, and elastomeric fibers, a fabric may be constructed that is primarily polyester-containing on one side while the opposite side of the fabric is primarily nylon-containing. The elastomeric fiber provides some level of stretch to the fabric, which may be useful for some wounds that require, for example, a dressing to be wrapped snugly around the wound site. The elastomeric fiber, in addition to providing conformability to the wound care device, also provides some level of softness to the device. Spandex is one non-limiting example of an elastomeric fiber and may be known by the tradename LYCRA®, which is available from INVISTA of Wichita, Kansas.

Additionally, it may be generally known to those skilled in the art that a knit polyester fabric tends to be hydrophobic, slow to absorb liquids, and generally exhibits little or no wicking of moisture. Since polyester is hydrophobic in nature, conventional wisdom would lead one to choose a hydrophilic natural fiber, such as cotton, or a hydrophilic synthetic fiber, such as nylon, as the wound contacting side of the wound care device. However, it was unexpectedly discovered that by placing a hydrophobic polyester containing surface against the wound site and a hydrophilic nylon containing surface away from the wound site, a unique one-way, directional flow of fluid away from the wound site was achieved.

The wound care device may be of any thickness, depending on the construction of the fabric. It may, however, be preferred that the thickness of the wound care device is between about 25 and about 60 mils. It may be more preferred that the wound care device is between about 35 and about 50 mils. It may be even more preferred that the wound care device is between about 38 and about 45 mils. It should be understood, and is exemplified herein, that thickness measurements may be increased when the wound care device also includes an antimicrobial finish on one or more surfaces of the wound care device.

While potentially preferred fabrics have been described, it is believed that any fiber or fabric that has been treated with the silver-based antimicrobial chemistry described herein is suitable for use within the present wound care device, as well as any of the above-mentioned substrate materials.

### Antimicrobial and Other Agents

The particular treatment used herein comprises at least one silver ion-releasing compound selected from the group consisting of silver ion exchange materials (e.g. zirconium phosphates and zeolites),

Other silver ion-containing materials may also be used. Various combinations of these silver-containing materials may be made if adjustments to the silver release rate over time are desired.

Generally, the silver-based compound is added in an amount from about 0.01% to about 60% by total weight of the particular finish composition; more preferably, from about 0.05% to about 40%; and most preferably, from about 0.1% to about 30%. The antimicrobial finish itself, including any desired binders, wetting agents, odor absorbing agents, leveling agents, adherents, thickeners, and the like, is added to the substrate in an amount of at least about 0.01% of the total device weight.

A binder material has been found useful in preventing the antimicrobial from flaking onto the wound. Preferably, this component is a polyurethane-based binding agent, although a wide variety of cationic, anionic, and non-ionic binders may also be used, either alone or in combination. Preferably, the binding agent is biocompatible such that it does not cause negative reactions in the wound. In essence, such binders provide durability by adhering the antimicrobial to the target substrate, such as fibers or fabrics, without negatively affecting the release of silver ions to the wound.

Total add-on levels of silver to the target substrate may be 20 ppm or higher. More preferably, total add-on levels of silver may be 200 ppm or higher. Although an upper boundary limit of silver add-on levels to the target substrate has not been determined, consideration of the manufacturing economics and the potential to irritate a sensitive wound site suggests avoiding excessive silver levels.

### Application of Antimicroblal and Other Agents to Substrate

Silver ion-containing compounds (such as ALPHASAN®, ZEOMIC®, or IONPURE®) are admixed in an aqueous dispersion with a binder to form a bath into which the target substrate is immersed. Other similar types of compounds that provide silver ions may also be utilized.

When specific polyurethane-based binder materials are utilized, the antimicrobial characteristics of the treated substrate are effective with regard to the amount of surface available silver that is released to kill bacteria, without altering the color of the treated substrate (that is, while substantially maintaining its original appearance). While it currently appears that the use of polyurethane-based binder resins are preferred due to their allowance of silver release and bio-neutral properties, in practice essentially any effective cationic, anionic, or non-ionic binder resin that is not toxic to the wound may be used.

An acceptable method of providing a durable antimicrobial silver-treated fabric surface is the application of a silver ion-containing compound and polyurethane-based binder resin from a bath mixture. This mixture of antimicrobial compound and binder resin may be applied through any technique as is known in the art, including spraying, dipping, padding, foaming, printing, and the like. By using one or more of these application techniques, a fabric may be treated with the antimicrobial compound and binder resin on only one side of the fabric (e.g. the wound contact surface of a wound care device), or it may be treated on both sides of the fabric.

The following examples further illustrate the present antimicrobial device but are not to be construed as limiting the invention as defined in the claims appended hereto. All parts and percents given in these examples are by weight unless otherwise indicated.

### Sample Creation and Evaluation

### A. Substrate Descriptlons

The fiber used in **Example 1** was a 70 denier 34 filament DACRON® polyester fiber.

The fabric used in **Examples 2A** - **2E** and **Example 2 Control** was a nonwoven fabric comprised of natural and synthetic fibers. The fabric weight is approximately 68 g/m². The fabric is manufactured and sold by Ahlstrom.

The fabric used in **Examples 3A - 3B** and **Example 3 Control** was a point-bonded nonwoven fabric, having a fabric weight of 130 g/m² and sold under the tradename EVOLON® by Firma Carl Freudenberg of Weinheim, Germany. Polyester fiber comprised about 65% of the fabric, and nylon 6,6 fiber comprised about 35% of the fabric. The fabric was not dyed.

The fabric used in **Examples 4A - 4D** and **Example 4 Control** was a nonwoven fabric made of 100% polyester having a weight of approximately 40 g/m². The fabric is sold under the tradename CELFIL® by Polimeros y Derivados. As purchased (prior to addition of an antimicrobial composition), the fabric contained optical brightening agents to enhance the fabric's brightness.

The fabric used for **Examples 5** - **8** was a jersey knit (circular knit), multi-polymer fabric sold by Milliken & Company. The fabric was single layer of fabric comprised of approximately 66% continuous filament polyamide yarn, 19% continuous filament polyester yarn, and 15% continuous filament spandex yarn. The polyamide yarn was comprised of 2 plies of 40 denier / 34 filament count nylon 6 fiber that was exposed to a texturing process prior to knitting. The polyester yarn was comprised of single ply 70 denier / 34 filament count fiber that was exposed to a texturing process prior to knitting. The spandex yarn was comprised of 55 denier / 3 filament count fiber. The fabric was knitted in such as manner as to give a distinct nylon side and a distinct polyester side. The polyester side of the fabric was exposed to a face-finishing process known as sanding.

### B. Antimicrobial Coating Formulations

Various aqueous dispersions of an antimicrobial finish include combinations of the following components:
- Antimicrobial ALPHASAN® RC2000 silver-based ion exchange compound, available from Milliken & Company of Spartanburg, SC
- Aqueous dispersion of nanosilver particles having an average particle size of between 20nm and 80nm, available from CIMA Nanotech of St. Paul, MN
- WITCOBOND® polyurethane binders available from Chemtura Corporation of Middlebury, CT
- LUBRIL QCJ, a hydrophilic polymer dispersion available from Roebuck Operations of Spartanburg, SC
- FREECAT MX, an aqueous white liquid consisting of a buffered magnesium salt available from Noveon Chemicals of Cleveland, OH

### EXAMPLE 1 (Reference)

A 70-denier, 34 filament DACRON® polyester fiber was used for Example 1. A solution was prepared according to the formulation in TABLE 1 and was applied to the polyester fiber using an Atlab finish applicator manufactured by Atlas Industries. Prior to testing, 12 strands of this fiber were hand twisted into a yarn about 5 cm in length. The resulting fiber had a 7.5% wt/wt content of ALPHASAN® RC2000. The ratio of ALPHASAN® RC 2000 to binder was 1:1.

**TABLE 1**

| **Component** | **Amount (grams)** |
|---|---|
| Water | 37 |
| Witcobond W-293 (polyurethane binder; 67% solids) | 1.5 |
| AlphaSan® RC 2000 (antimicrobial agent, 10% Ag) | 1.5 |

### EXAMPLES 2A-2E (Reference)

The AHLSTROM® nonwoven fabric described above was coated using the formulations shown below in **TABLE 2**. Examples 2A through 2E were prepared using the following steps:
(a) The coating solution was prepared at room temperature via stirring the components listed below in a container for approximately one hour; and
(b) the fabric was dipped in a bath, squeezed via nip rollers, and dried in an oven at around 350 °F for between two and three minutes.

A Control sample (Example 2 Control) was also prepared in a water-only solution, which was exposed to the same process conditions as Examples 2A - 2E.

**TABLE 2**

| *Formulations for Examples 2A* - *2E* | | | | | | | |
|---|---|---|---|---|---|---|---|
| | **Components (grams)** | | | | | | |
| **Sample ID** | **Water** | **AlphaSan® RC 2000** | **Witcobond W-293** | **Lubril QCX** | **Freecat MX** | % **Active AlphaSan® (wt/.wt%)** | **Ratio of AlphaSan® RC 2000 to Binder** |
| Example 2A * | 365.3 | 100.0 | 15.0 | 9.8 | 10.0 | 20.7% | 6.6 : 1 |
| Example 2B * | 409.5 | 51.2 | 19.1 | 10.0 | 10.2 | 8.2% | 2.7 : 1 |
| Example 2C * | 417.9 | 25.0 | 37.3 | 9.8 | 10.0 | 4.2% | 0.67 : 1 |
| Example 2D * | 398.3 | 5.1 | 76.4 | 10.0 | 10.0 | 1.1% | 0.067 : 1 |
| Example 2E * | 477.7 | 5.0 | 7.5 | 9.78 | 0.08 | 1.0% | 0.67 : 1 |

| | | | | | | | |
|---|---|---|---|---|---|---|---|
| NOTE: The weight/weight % is calculated by dividing the weight of antimicrobial agent as determined by analytical procedure by the weight of the dry coated fabric. *Reference | | | | | | | |

### EXAMPLES 3A - 3B (Reference)

The EVOLON® nonwoven fabric described above was coated using the formulations shown below in **TABLE 3**. These Examples were prepared via the same process used to create the finished fabrics of Example 2. A Control sample (Example 3 Control) was also prepared in a water-only solution, which was exposed to the same process conditions as Examples 3A-3B.

**TABLE 3**

| *Formulations for Examples 3A - 3B* | | | | | | | |
|---|---|---|---|---|---|---|---|
| | **Components (grams)** | | | | | | |
| **Sample ID** | **Water** | **AlphaSan® RC 2000** | **Witcobond W-293** | **Lubtil QCX** | **Freecat MX** | **% Active Alphasan® (wt./wt.%)** | **Ratio of AlphaSan® RC 2000 to Binder** |
| Example 3A * | 961.1 | 444.8 | 331.7 | 173.9 | 88.9 | 18.2% | 1.34 : 1 |
| Example 3B * | 1740.3 | 111.2 | 82.9 | 43.5 | 22.2 | 2.2% | 1.34 : 1 |

| | | | | | | | |
|---|---|---|---|---|---|---|---|
| NOTE: The weight/weight % is calculated by dividing the weight of antimicrobial agent by the weight of the coated fabric. *Reference | | | | | | | |

### EXAMPLES 4A - 4D (Reference)

The CELFIL® nonwoven fabric described above was coated using the formulations shown below in **TABLE 4**. These Examples were prepared via the same process used to create the finished fabrics of Example 2. A Control sample (Example 4 Control) was also prepared in a water-only solution, which was exposed to the same process conditions. The fabric used to create Examples 4A - 4D and Example 4 Control included an optical brightener to enhance its brightness.

**TABLE 4**

| *Formulations for Examples 4A - 4D* | | | | | | | |
|---|---|---|---|---|---|---|---|
| | **Components (grams)** | | | | | | |
| **Sample ID** | **Water** | **AlphaSan® RC 2000** | **Witcobond W-293** | **Lubril QCX** | **Freecat MX** | **% Active Alphasan® (wt./wt%)** | **Ratio of AlphaSan® RC 2000 to Binder** |
| Example 4A * | 1707.1 | 175.5 | 65.5 | 34.3 | 17.5 | 16.0% | 2.7 : 1 |
| Example 4B * | 456.4 | 25.0 | 18.7 | 0 | 0 | 9.9% | 1.34 : 1 |
| Example 4C * | 288.4 | 25.0 | 186.6 | 0 | 0 | 5.0% | 0.13 : 1 |
| Example 4D * | 457.7 | 5.0 | 37.3 | 0 | 0 | 1.2% | 0.13 : 1 |

| | | | | | | | |
|---|---|---|---|---|---|---|---|
| NOTE: The weight/weight % is calculated by dividing the weight of antimicrobial agent as determined by analytical procedure by the weight of the dry coated fabric. *Reference | | | | | | | |

### EXAMPLES 5A and 5B

The multi-polymer fabric described above was coated using the formulations shown below in **TABLE 5**. These Examples were prepared via the same process used to create the finished fabrics of Example 2. In Example 5A, an optimal brightener was included as part of the material (before application of the antimicrobial composition). Example 5B did not include an optical brightener.

Two Control samples (Example 5A Control, which contained an optical brightener, and Example 5B Control, which did not contain an optical brightener) were also prepared in a water-only solution, which was exposed to the same process conditions as Examples 5A and 5B.

Measurements of the Example fabrics and the Control fabrics were made from both the nylon side of the material and the polyester side of the material.

**TABLE 5**

| *Formulations for Examples 5A - 5B* | | | | | | |
|---|---|---|---|---|---|---|
| | **Components (grams)** | | | | | |
| **Sample ID** | **Water** | **AlphaSan® RC 2000** | **Witcobond W-293** | **Optical Brightener** | **% Active Alphasan® (wt./wt%)** | **Ratio of AlphaSan® RC 2000 to Binder** |
| Example 5A | 3083.4 | 705.9 | 210.7 | Yes | 14.4 | 3.35 : 1 |
| Example 5B | 2083.4 | 705.9 | 210.7 | No | 20.1 | 3.35 : 1 |

The weight/weight % is calculated by dividing the weight of antimicrobial agent as determined by analytical procedure by the weight of the dry coated fabric.

### EXAMPLE 6 - Nanoparticle Silver (Reference)

The fabric used in Example 6 was the multi-polymeric fabric of Example 5. This Example was prepared using the same process used to create the fabrics of Example 2. The formulation for this Example is shown in **TABLE 6.** The ratio of nanoparticle silver to binder was 0.66 : 1.

**TABLE 6**

| *Nanoparticle Silver Formulation* | |
|---|---|
| **Component** | **Amount (grams)** |
| Water | 191 |
| Witcobond 290H (polyurethane binder; 62% solids) | 5.3 |
| Cima NanoTech product no. AB120-1 (antimicrobial agent) | 3.5 |

### EXAMPLES 7A - 7F

The fabric used in **Example 7** was the multi-polymeric fabric used in Example 5. They were dyed with one of the following pastel dye colors and concentrations.
**7A:** Pastel blue color; full dye concentration
**7B:** Pastel blue color; half dye concentration
**7C:** Pastel green color; full dye concentration
**7D:** Pastel green color; half dye concentration
**7E:** Pastel purple color; full dye concentration
**7F:** Pastel purple color; half dye concentration

**Examples 7A - 7F** were coated with the same antimicrobial finish (formulation shown in **TABLE 7**). Control samples, corresponding to each of the dyed samples and having the same dye color and amount, were also created and subjected to the same processing conditions. The ratio of ALPHASAN® RC 2000 to binder was 3.33 : 1.

**TABLE 7**

| *Formulation used for Examples 7A* - *7F* | |
|---|---|
| **Component** | **Amount (grams)** |
| Water | 1388.1 |
| Witcobond W-293 (polyurethane binder) | 141.2 |
| AlphaSan® RC 2000 (antimicrobial agent, 10% Ag) | 471.2 |

### EXAMPLE 8A

A solution was prepared according to the formulation shown below and was applied to the jersey knit fabric of Example 5 (described previously). The fabric was passed through a bath containing the antimicrobial formulation and subsequently through squeeze rollers to achieve a wet pick-up of about 85%. The fabric was then dried in a tenter frame to remove excess liquid.

The solution yielded a percent active ALPHASAN® RC 2000 content of 16 weight percent based on the weight of the fabric, as determined by the Total ALPHASAN® RC 2000 Content Test described herein. The ratio of ALPHASAN® RC 2000 to binder was 1.8 : 1.

**TABLE 8A**

| *Formulation used for Example 8A* | |
|---|---|
| **Component** | **Amount (pounds)** |
| Water | 300.0 |
| Witcobond® UCX-281F (polyurethane binder, 40% solids) | 52.9 |
| AlphaSan® RC 2000 (antimicrobial agent, 10% Ag) | 95.3 |

### EXAMPLE 8B

A solution was prepared according to the formulation shown below and was applied to the jersey knit fabric of Example 5 (described previously). The fabric was passed through a bath containing the antimicrobial formulation and subsequently through squeeze rollers to achieve a wet pick-up of about 85%. The fabric was then dried in a tenter frame to remove excess liquid.

The solution yielded a percent active ALPHASAN® RC 2000 content of 17 weight percent based on the weight of the fabric, as determined by the Total ALPHASAN® RC 2000 Content Test described herein. The ratio of ALPHASAN® RC 2000 to binder was 3.35: 1.

**TABLE 8B**

| *Formulation used for Example 8B* | |
|---|---|
| **Component** | **Amount (pounds)** |
| Water | 328.0 |
| Witcobond® 293 (polyurethane binder) | 28.0 |
| AlphaSan® RC 2000 (antimicrobial agent, 10% Ag) | 94.0 |

Each of **Examples 8A** and **8B**, when applied to the jersey knit fabric, result in a wound care device comprising approximately 16% polyester fiber, 53% nylon fiber, 12% spandex fiber, 15% antimicrobial agent, and 4% binding agent.

Based on the exemplary formulations provided above and the test results provided below, it is contemplated that one preferred embodiment of the present wound care device is a substrate having a non-electrically conductive finish, in which the finish comprises at least one silver ion-releasing compound and at least one binder material, the silver ion-releasing compound being present in an amount that is at least 35% more than the amount of the binder material. The substrate, as mentioned previously, is preferably a fiber, fabric, or alginate, and is most preferably a fabric.

In an alternate embodiment, which may be preferred for moisture management purposes, a fabric constructed to have a hydrophobic side and a hydrophilic side (such as in **Examples 5-8**) may be used, in which the hydrophobic side is used as the wound contact side and the hydrophilic side is used as the wound fluid reservoir side. Fluids are drawn through the contact side (away from the wound) and are held in the reservoir side. In this instance, although the presence of a silver ion-releasing compound may be beneficial in promoting wound healing, it is not required.

### C. Comparative Sample Descriptions

Several commercially available silver-containing wound care devices were also purchased for evaluation. These textile-based wound care devices are notated as **Comparative Examples A - M** below and include a wide variety of wound dressing combinations.

**COMPARATIVE EXAMPLE A -** "ACTISORB® 220", a multi-component nonwoven wound care device comprised of a highly porous, silver impregnated charcoal cloth (containing 220 mg of silver per 100 g of activated charcoal cloth) sandwiched between two nylon nonwoven layers; available from Johnson & Johnson of Somerville, New Jersey.

**COMPARATIVE EXAMPLE B -** °ACTICOAT® 5", a three layered wound care device consisting of three layers-a layer of polyethylene film, a middle layer of rayon/polyester blend nonwoven fabric, and a second layer of film; nano-crystalline silver particles are deposited onto the film layers; available from Smith and Nephew of Largo, Florida.

**COMPARATIVE EXAMPLE C -** "ACTICOAT® 7", a five-layered wound care device consisting of an inner sheet of polyethylene film treated with nano-crystalline silver sandwiched between two layers of untreated rayon fabric; this composite structure is further sandwiched between two layers of polyethylene film treated with nano-crystalline silver; available from Smith and Nephew of Largo, Florida.

**COMPARATIVE EXAMPLE D -** "Silverlon", a silver-plated nylon fabric; available from Argentum Medical, LLC of Lakemont, Georgia.

**COMPARATIVE EXAMPLE E -** "AQUACEL® Ag", a silver-impregnated sodium carboxymethyl cellulose hydrofiber having 1.2% silver; available from Convatec, a Bristol-Myers-Squibb Company of England.

**COMPARATIVE EXAMPLE F** - "AQUACEL®", a hydrofiber dressing; available from Convatec, a Bristol-Myers-Squibb Company of England.

**COMPARATIVE EXAMPLE G -** "LYOFOAM®", a sterile, non-adherent, absorbent dressing; available from Convatec, a Bristol-Myers-Squibb Company of England.

**COMPARATIVE EXAMPLE H** - "Foam Dressing", a non-adherent foam; available from 3M of St. Paul, Minnesota.

**COMPARATIVE EXAMPLE I** - "Woven Gauze Pad", a medical dressing manufactured for Dynarex Corporation of Orangeburg, New York.

**COMPARATIVE EXAMPLE J -** "MIRASORB™ Gauze", a nonwoven gauze; available from Ethicon, Inc., a division of Johnson & Johnson of Somerville, New Jersey.

**COMPARATIVE EXAMPLE K -** "EVOLON®", a nonwoven fabric comprised of multi-component fibers; the multi-component fibers are comprised of individual nylon and polyester fibers that have been longitudinally split into their individual components by hydroentanglement; the fabric is described in US Patent Nos. 5,899,785 and 5,970,583 both of which are assigned to Firma Carl Freudenberg of Weinheim, Germany and which are incorporated entirely herein by reference. This is the same fabric used in Example 3.

**COMPARATIVE EXAMPLE L- "**VERSALON™**",** nonwoven medical sponges; available from Kendall and trademark of Tyco Healthcare Group LP.

**COMPARATIVE EXAMPLE M** - "CONTREET® F", a polyurethane foam having ALPHASAN® RC 2000 silver throughout the polymer matrix; available from Coloplast A/S.

### D. Examples Testing and Evaluation

Each of the above examples was tested for a variety of characteristics as will be described below. Further, some of the commercially available products (referred to as Comparative Examples A - M and described above) were also tested for comparison with the present antimicrobial wound care substrates. The testing procedures will be described in detail as follows. However, a listing of the tests used is found below.
- **Test 1.**: Total ALPHASAN® RC 2000 Content Test (Ashing Technique)
- **Test 2.**: Zone of Inhibition Test (Kirby-Bauer Agar Diffusion Assay)
- **Test 3.**: Quantitative Log Reduction Test (Modified AATCC Method 100)
- **Test 4.**: Wavelength / Reflectance Evaluation
- **Test 5.**: Whiteness / Yellowness Evaluation
- **Test 6.**: Color Evaluation: Lightness/Darkness, Yellow/Blue, Red/Green
- **Test 7.**: Comparison of Magnitude of Color Difference between Sample and Reference Tile
- **Test 8.**: Color Stability Test
- **Test 9.**: Fluid Transport Test (Internally developed method)
- **Test 10.**: Tensile Strength Test (ASTM D 5034)
- **Test 11.**: Conductivity/Resistivity Test (AATCC Test Method 76)
- **Test 12.**: Thickness Test (ASTM D 1777-96)

### TEST 1 : Total ALPHASAN® Content

The amount of ALPHASAN® antimicrobial incorporated into or onto an article can be determined by measurement of elements unique to the antimicrobial compound. For ALPHASAN® antimicrobial, the two elements of highest abundance are silver or zirconium. Because zirconium is more abundant in the ALPHASAN® antimicrobial product and is easier to measure, it is preferable to use zirconium as the signature element for determining the level of ALPHASAN® antimicrobial in an article. The amount of ALPHASAN® antimicrobial incorporated into or onto the wound care device was determined using the following ashing technique.

A sample of fabric (weighing approximately 1 gram but with weight measured to four significant digits) was placed in a clean, dry ceramic crucible which had been weighed. The crucible containing the fabric sample was placed in a muffle furnace whose temperature ramped up at 3 °C/minute to 750 °C. The temperature was then held at 750 °C for four hours. The system was then cooled and the crucible transferred to a desiccator in which it was allowed to reach an equilibrium temperature. The crucible was then weighed. This provides the percent solids of inorganic constituents.

The fabric sample was then ground in the ceramic crucible to obtain a uniform sample. Approximately 0.05g weight (again measured to four significant digits) was then taken from the ceramic crucible and placed in a platinum crucible. Four milliliters of 50% HNO₃, followed by 15-20 drops of 48% HF, were added to the crucible. The crucible was heated over a hot plate until the sample completely dissolved. The sample solution was then transferred to a 100 mL volumetric flask.

The crucible was then rinsed with 5% HNO₃, with the rinse solution being added to the flask. The solution was diluted to the 100 mL mark with 5% HNO₃. The dilute solution was transferred to a polyethylene storage container. Analysis for the desired active ingredient (in this case, zirconium) was performed using an Inductively Coupled Plasma Optical Emission Spectrometer device (e.g., a Perkin Elmer Optima 4300DV). Calculations are apparent to one skilled in the art. The amount of ALPHASAN® RC2000 present on the wound care device is provided as a weight percent based on the weight of the fabric. The results are shown in **TABLE 9**.

**TABLE 9**

| *Total ALPHASAN*® *RC2000 Content of Inventive Wound Care Device* | |
|---|---|
| **Sample ID** | **Percent ALPHASAN® RC2000** |
| Example 1 * | 7.5 |
| Example 2A * | 20.7 |
| Example 2B * | 8.2 |
| Example 2C * | 4.2 |
| Example 2D * | 1.1 |
| Example 2E * | 1.0 |
| Example 3A * | 18.2 |
| Example 3B * | 2.2 |
| Example 4A * | 16.0 |
| Example 4B * | 9.9 |
| Example 4C * | 5.0 |
| Example 4D * | 1.2 |
| Example 5A | 14.4 |
| Example 5B | 20.1 |
| Example 8A | 16 |
| Example 8B | 17 |

| | |
|---|---|
| *Reference | |

### TEST 2: Zone of Inhibition Test (Kirby-Bauer Agar Diffusion Assay)

Several Gram-positive and Gram-negative bacteria as well as fungi (yeast) were chosen to illustrate the antimicrobial efficacy of the inventive wound care device. Gram-positive bacteria include, for example and without limitation, *Staphylococcus aureus, Clostridium perfringens, Enterococcus faecium* and *Bacillus cereus.* Gram-negative bacteria include, for example and without limitation, *Klebsiella pneumoniae, Escherichia coli, Acinetobacter baumannii, Enterobacter cloacae, Proteus mirabilis, and Pseudomonas aeruginosa.* Fungi, such as yeast, include for example, *Candida albicans* and *Saccharomyces cerevisiae*. Many of these organisms were selected to demonstrate the antimicrobial efficacy of the Examples below. However, it should be understood to be within the scope of this invention that similar results would be obtained against other bacteria and fungi.

The Zone of Inhibition (ZOI) Test based on the Kirby-Bauer Agar Diffusion Assay provides both a qualitative (level of growth underneath sample) and quantitative (size of zone in millimeters) assessment of the performance of an antimicrobial agent incorporated into a wound dressing. The level of growth underneath the sample can be rated from confluent ("no activity") to spotty or isolated ("bacteriostatic") to nil ("bactericidal"). If reduced growth is observed underneath the sample for a particular microorganism when compared to an untreated control dressing, that microorganism is considered sensitive and the antimicrobial agent is effective (i.e., is bacteriostatic). The magnitude of the zone of inhibition, if one is observed, is a measure of both the inherent efficacy of the agent and the diffusion of the agent through the nutrient agar matrix. This zone of inhibition assay can be used to measure the efficacy of the dressings in a simulated clinical application by subjecting the dressings to multiple insults of a high level of bacteria over a multiple-day period. For purposes of discussion herein, a substrate is considered to have "effective antimicrobial" properties if it produces a ZOI against bacteria of at least 1 mm after two successive exposures.

Examples 1 - 5 and Comparative Examples A - E were tested against one or more of *Staphylococcus aureus* ATCC #6538 using a standard zone of inhibition test based on the Kirby-Bauer Agar-Diffusion Assay. The procedure is described in the report "Antibiotic Susceptibility Testing by a Standardized Single Disc Method" written by A.W. Bauer, W.M. Kirby, and M. Truck and published in the American Journal of Clinical Pathology 1966; Volume 45, page 493.

Examples 1 - 5 and Comparative Examples A - E were tested according to the following procedure. Petri dishes containing Diagnostic Sensitivity Test (DST) agar were inoculated via spreading with 0.5 mL of a diluted overnight culture of approximately 5E+05 cells/mL of the test organism into 100mM Na/K phosphate buffer. An approximately 1 inch by 1 inch piece of the Example fabric was then placed at the center of each agar plate. The agar plates were incubated for 24 hours at 37 °C, after which the level of efficacy was determined. To simulate repeated exposure of the dressings to microbes, the dressings were removed from the incubated agar plate, placed onto freshly inoculated agar plates, and incubated another 24 hours. This process was repeated for up to 7 days.

In some cases, an untreated (i.e., without antimicrobial finish) fabric was also tested according to this method. The Control sample was a MEDISPONGE® polyurethane foam available from Lendell, an antimicrobial-free substrate.

The Zone of Inhibition Test was conducted to determine the antimicrobial activity of the Examples against *Staphylococcus aureus.* The results, which are shown in TABLE 10, represent an average of four measurements for each sample (one from each of the four sides of the square sample).

**TABLE 10**

| *Antimicrobial Activity of Examples 1-5 and Comparative Examples A-E Against Staphylococcus aureus ATCC #6538 as Determined By Zone of Inhibition Method* | | | | | | | |
|---|---|---|---|---|---|---|---|
| **Sample** | **Average Day 1 Zone (mm)** | **Average Day 2 Zone (mm)** | **Average Day 3 Zone (mm)** | **Average Day 4 Zone (mm)** | **Average Day 5 Zone (mm)** | **Average Day 6 Zone (mm)** | **Average Day 7 Zone (mm)** |
| Example 1 * | 3.0 | n/d | n/d | n/d | n/d | n/d | n/d |
| Example 2A * | 9 | 5 | 7 | 7 | 5 | 5 | 4 |
| Example 2B * | 10 | 6 | 6 | 5 | 2 | 0 | 0 |
| Example 2C * | 9 | n/d | 5 | 3 | 1 | 0 | 0 |
| Example 2D * | 7 | 1 | 0 | 0 | 0 | 0 | 0 |
| Example 2 - Control | 0 | 0 | 0 | 0 | 0 | 0 | 0 |
| Example 3A * | 4 | 3 | 3 | 3 | 3 | 2 | 4 |
| Example 3B * | 4 | 3 | 3 | 3 | 2 | 2 | 5 |
| Example 3 - Control | 0 | 0 | 0 | 0 | 0 | 0 | 0 |
| Example 4A * | 7 | 5 | 4 | 4 | n/d | n/d | n/d |
| Example 4B * | 3 | 3 | 1 | 0 | n/d | n/d | n/d |
| Example 4C * | 0 | 0 | 0 | 0 | n/d | n/d | n/d |
| Example 4D * | 0 | 0 | 0 | 0 | n/d | n/d | n/d |
| Example 4 - Control | 0 | 0 | 0 | 0 | 0 | 0 | 0 |
| Example 5B | 5 | 4 | 4 | 4 | 4 | 3 | 4 |
| Example 5 - Control | 0 | 0 | 0 | 0 | 0 | 0 | 0 |
| Comparative Ex. A | 0 | 0 | 0 | 0 | n/d | n/d | n/d |
| Comparative Ex. B | 4 | 4 | 4 | 5 | 5 | 4 | 4 |
| Comparative Ex. C | 6 | 8 | 7 | 7 | 7 | 5 | 9 |
| Comparative Ex. D | 5 | 6 | 5 | 5 | n/d | n/d | n/d |
| Comparative Ex. E | 8 | 5 | 6 | 6 | 7 | 4 | 3 |

| | | | | | | | |
|---|---|---|---|---|---|---|---|
| NOTE: "n/d" indicates that the value was not determined. *Reference | | | | | | | |

The results demonstrate that the present **Examples 1-5,** all of which contained ALPHASAN® RC 2000, were antimicrobially active against *Staphylococcus aureus.* The control samples, which contained no antimicrobial agent, did not demonstrate antimicrobial activity against any of the bacteria.

**Example 5B Control, Example 8B, and Comparative Examples C** and **D** were tested to determine their antimicrobial activity against several microbes using a modified version of the Kirby-Bauer Susceptibility Test. A brief description of the test method is included below. A full description of the test method may be found in the following document: National Committee for Clinical Laboratory Studies (NCCLS) M2-A8: Performance Standards for Antimicrobial Disk Susceptibility Tests; Approved Standard-Eighth Edition; 2003.

An overnight culture of the test microbe was diluted into saline (0.85% NaCl) to a concentration of 10⁶ cells/ml. Petri dishes containing Diagnostic Sensitivity Test (DST) Agar were inoculated with 0.25ml of the cell suspension and incubated for 1 hour. A sample (15 mm diameter circle) of each wound care device was then placed at the center of the agar plate. The agar plate was incubated for 24 hours at 37°C. After measuring the extent of the zones (in mm), the samples were transferred to a fresh DST plate inoculated with the same microbe. The process was repeated for three days (total). The results of the Zone of Inhibition Test for each sample are shown in **TABLE 11**.

**TABLE 11**

| *Zone of Inhibition for Inventive and Comparative Wound Care Devices* | | | | | | | | | |
|---|---|---|---|---|---|---|---|---|---|
| Microbe (ATCC#) | Zone of Inhibition (mm) | | | | | | | | |
| | Example 8B (ALPHASAN® RC 2000) | | | Comparative Example C (ACTICOAT® 7) | | | Comparative Example D (SILVERLON®) | | |
| | Day 1 | Day 2 | Day 3 | Day 1 | Day 2 | Day 3 | Day 1 | Day 2 | Day 3 |
| *Acinetobacter baumannii* (#19606) | 5.5 | 5 | 4 | 6 | 5 | 4 | 5.5 | 3.5 | 0.5 |
| *Enterobacter cloacae* (#13047) | 1.5 | 1 | 1 | 1 | 1 | 0.5 | 1 | 1 | 0 |
| *Enterococcus faecalis* (#51299) (VRE) | 3.5 | 2.5 | 1.5 | 4.5 | 3 | 2 | 3.5 | 2.5 | 1.5 |
| *Enterococcus faecium* (#19434) | 5.5 | 3.5 | 6 | 5.5 | 3.5 | 6 | 6 | 2 | 4.5 |
| *Escherichia coli* (#10536) | 4.5 | 4 | 2 | 4.5 | 4 | 2.5 | 4 | 2 | 0 |
| *Escherichia coli* (#8739) | 5 | 4.5 | 3 | nd | nd | nd | nd | nd | nd |
| *Klebsiella pneumoniae* (#4352) | 5.5 | 3 | 3 | 4.5 | 3.5 | 3 | 5 | 2 | 0 |
| *Proteus mirabilis* (#25933) | 5 | 2 | 0 | 4 | 2.5 | 2 | 3.5 | 0 | 0 |
| *Proteus vulgaris* (#8427) | 3.5 | 3 | 2.5 | 3 | 3 | 3 | 2.5 | 1 | 0 |
| *Staphylococcus aureus* (#6538) | 6 | 5 | 3 | 5 | 5 | 4 | 5 | 5 | 4 |
| *Staphylococcus aureus* (#43300) (MRSA) | 4.5 | 3 | 2.5 | 3.5 | 2.5 | 2.5 | 4 | 2.5 | 2.5 |
| *Staphylococcus epidermidis* (#12228) | 8 | 4 | 3.5 | 7 | 4 | 4 | 7 | 2 | 1 |
| *Staphylococcus epidermidis* (#51625) (MRSE) | 7.3 | 5.5 | 4.5 | 7 | 6.5 | 5 | 7 | 6 | 4.5 |
| *Candida albicans* (#10231) | 6 | 4 | 4 | 7 | 4 | 4 | 6 | 2 | 0 |
| *Saccharomyces cerevisiae* (#9763) | 4 | 5 | 5.5 | 4 | 4 | 1.5 | 3 | 1.5 | 0 |

| | | | | | | | | | |
|---|---|---|---|---|---|---|---|---|---|
| nd = not determined | | | | | | | | | |

The test results in **TABLE 11** illustrate that the inventive wound care device (**Example 8B**) exhibits considerable efficacy against several types of Gram-positive and Gram-negative bacteria, as well as against various fungi (yeast). **Example 5B Control** (no antimicrobial treatment) did not exhibit any efficacy (data not shown).

### TEST 3: Quantitative Log Reduction Test (Modified AATCC Method 100)

**Example 2, Example 3,** and **Comparative Examples C** and **E** were tested for antimicrobial performance. Efficacy against bacteria was assessed using a modified version of AATCC Method 100-1999 as described below.

Portions (approximately 0.5 g) of each fabric were placed in glass vials and exposed to two types of bacteria-*Staphylococcus aureus* ATCC #6538 (0.5 mL of 1.31E+06 cells/mL) or *Pseudomonas aeruginosa* ATCC #12055 (0.5 ml of 2.63E+05 cells/mL)-each of which was suspended in a solution of 2% Bovine Serum Albumin in 0.85% NaCl for 18 - 22 hours at 37 °C. After incubation, the samples were washed to remove attached cells. The number of viable cells in the wash solution was quantified using a microtiter plate-based "Most Probable Number" assay. The results are shown in **TABLE 12**. Negative values, as shown for the Control, indicate bacterial growth.

**TABLE 12**

| *Efficacy of Antimicrobial Wound Care Devices As Determined By Log Reduction Kil Values* | | |
|---|---|---|
| **Sample ID** | ***S*. *aureus*** | ***P*. *aeruginosa*** |
| Example 2A * | 2.94 | n/d |
| Example 2B * | 2.57 | n/d |
| Example 2C * | 1.57 | n/d |
| Example 2D * | 3.57 | n/d |
| Example 2 - Control | -1.06 | n/d |
| Example 3A * | 1.36 | 1.69 |
| Example 3B * | 2.02 | 2.21 |
| Example 3 - Control | -0.89 | -2.06 |
| Comparative Example C | 4.34 | 4.31 |
| Comparative Example E | 0.8 | n/d |

| | | |
|---|---|---|
| n/d = not determined *Reference | | |

**Examples 5B Control, Example 8B**, and **Comparative Examples C** and **M** were tested for antimicrobial efficacy against *Staphylococcus aureus* ATCC #6538. The quantitative reduction of bacteria after exposure to the treated samples versus untreated fabric was assessed using a modified version of AATCC Method 100-1999, "Antibacterial Finishes on Textile Materials: Assessment of." The test procedure is described below.

Samples (about 40mm in diameter) were placed in sterile 60 ml plastic jars and exposed to bacteria (0.4ml of 10⁵ cells/ml) suspended in (a) simulated wound fluid (prepared as previously described) and 2% Bovine Serum Albumin and (b) simulated wound fluid (prepared as previously described) and 20% Bovine Serum. The jars were tightly capped and incubated for 22 hours at 37°C. The 0.4ml volume was determined to completely wet the wound dressing samples without excess fluid in the container. **Example 3** was inoculated on the polyester side of the wound care device. After incubation for 24 hours at 37°C, the samples were covered with 10ml of Tryptic Soy Broth with surfactants and cysteine to deactivate any residual silver and vortexed to remove attached cells. The number of viable cells in the wash solution was quantified using a microtiter plate-based "Most-Probable Number" assay. The level of viable cells recovered from the treated samples was compared to the number of cells recovered from untreated fabric and the percent reduction calculated. The results are shown in **TABLE 13**.

**TABLE 13**

| *Percent Reduction of Inventive and Comparative Wound Care Devices Compared to Untreated Fabric Against Staphylococcus aureus* | | |
|---|---|---|
| **Sample ID** | **Percent Reduction of *S. aureus* (SWF and 2% Bovine Serum Albumin)** | **Percent Reduction of *S*. *aureus* (SWF and 20% Bovine Serum)** |
| Example 5B Control (no antimicrobial agent) | 0.00 | 0.00 |
| Example 8B (ALPHASAN® RC 2000) | 99.96 | 99.75 |
| Comparative Example C (ACTICOAT® 7) | 99.99 | 99.99 |
| Comparative Example M (CONTREET® F) | 94.13 | 99.69 |

The results in **TABLE 13** indicate that over 99% of viable bacteria were reduced after 24 hours of contact with the inventive wound care device (**Example 8B**) compared to similar fabric without the antimicrobial treatment **(Example 5B Control).** For this testing, *Staphylococcus aureus* was selected as the representative microbe. However, it should be understood to be within the scope of this invention that Example **8B** would exhibit similar antimicrobial efficacy against other Gram-positive and Gram-negative bacteria and against fungi such as *C*. *albicans*.

### EVALUATING COLOR:

### PRESENT EXAMPLE DEVICES VS. COMPARATIVE SAMPLES

As has been previously discussed, providing an essentially white-colored, antimicrobial wound care device is desirable for a number of reasons. First, the antimicrobial properties of the device will facilitate the healing of the wound. This benefit has been described in detail herein. Secondly, by providing an essentially white-colored device, the health care provider and/or user are better able to monitor the exudates from the wound for signs of color change that could indicate a problem.

The wound care device of the present invention exhibits an unusual whiteness when compared to competitive silver-containing antimicrobial devices. This is, in part, due to the specific silver ion-releasing mechanism of the present finish. As will be discussed herein, the white color of the present wound care device is substantially consistent despite the add-on level of the silver-containing antimicrobial agent and is maintained over its shelf-life. **Examples 2-7,** as well as **Comparative Examples A - E,** were evaluated for color using various measurements, as will be described herein.

### TEST 4: Wavelength / Reflectance Evaluation

**Examples 2A - 2E, 3A - 3B, 4A - 4D, 5A - 5B, 6,** and **7A - 7F** were evaluated for color by measuring their reflectance across a given span of wavelengths corresponding to the visible spectrum (i.e., 360 nm - 750 nm). Control samples (i.e., fabrics containing no antimicrobial) for each of the present Examples were also evaluated. This evaluation was accomplished via use of a GretagMacbeth Color Eye 70001 spectrophotometer with a D-65 light source and a 10° observation angle. The **Comparative Examples** were also evaluated, using the same equipment. The reflectance data for **Examples 2** - 5 and the **Comparative Examples** is shown in **TABLES 14** - **18** below. This data was used to calculate Stensby Index for Whiteness and other comparisons below. Note that a higher reflectance percent indicates a lighter/whiter color.

It should also be noted that reflectance data, although not shown, was generated for **Examples 6** and **7A - 7F** and serves as the basis for the other color measurements of those Examples as will follow.

**TABLE 14**

| *Reflectance of Examples 2A - 2D and Ex. 2 Control* | | | | | |
|---|---|---|---|---|---|
| **Wavelength (nm)** | **Example * 2A (20.7%)** | **Example * 2B (8.2%)** | **Example * 2C (4.2%)** | **Example * 2D (1.1%)** | **Ex. 2 Control (0%)** |
| 360 | 62.066 | 50.841 | 55.029 | 48.954 | 51.407 |
| 370 | 68.091 | 61.149 | 62.708 | 56.198 | 63.494 |
| 380 | 72.152 | 67.144 | 68.933 | 62.251 | 70.499 |
| 390 | 75.398 | 71.047 | 74.110 | 67.510 | 74.569 |
| 400 | 78.111 | 74.093 | 77.822 | 71.604 | 77.448 |
| 410 | 80.473 | 76.755 | 80.397 | 74.683 | 79.681 |
| 420 | 82.628 | 79.207 | 82.360 | 77.209 | 81.532 |
| 430 | 84.419 | 81.338 | 83.862 | 79.293 | 83.010 |
| 440 | 85.974 | 83.181 | 85.157 | 81.112 | 84.295 |
| 450 | 87.339 | 84.760 | 86.263 | 82.703 | 85.451 |
| 460 | 88.563 | 86.118 | 87.235 | 84.135 | 86.465 |
| 470 | 89.669 | 87.329 | 88.118 | 85.428 | 87.385 |
| 480 | 90.627 | 88.389 | 88.855 | 86.598 | 88.172 |
| 490 | 91.365 | 89.216 | 89.421 | 87.526 | 88.785 |
| 500 | 92.090 | 90.015 | 89.987 | 88.436 | 89.406 |
| 510 | 92.582 | 90.586 | 90.391 | 89.096 | 89.840 |
| 520 | 92.962 | 91.029 | 90.698 | 89.610 | 90.192 |
| 530 | 93.273 | 91.407 | 90.960 | 90.053 | 90.515 |
| 540 | 93.563 | 91.781 | 91.242 | 90.459 | 90.821 |
| 550 | 93.742 | 92.034 | 91.406 | 90.752 | 91.010 |
| 560 | 93.909 | 92.252 | 91.571 | 91.029 | 91.193 |
| 570 | 94.030 | 92.424 | 91.694 | 91.254 | 91.326 |
| 580 | 94.155 | 92.584 | 91.824 | 91.462 | 91.472 |
| 590 | 94.270 | 92.733 | 91.968 | 91.648 | 91.632 |
| 600 | 94.349 | 92.848 | 92.082 | 91.833 | 91.768 |
| 610 | 94.338 | 92.869 | 92.100 | 91.903 | 91.812 |
| 620 | 94.439 | 92.991 | 92.222 | 92.065 | 91.938 |
| 630 | 94.482 | 93.082 | 92.304 | 92.184 | 92.029 |
| 640 | 94.585 | 93.212 | 92.435 | 92.361 | 92.175 |
| 650 | 94.608 | 93.258 | 92.482 | 92.442 | 92.234 |
| 660 | 94.685 | 93.370 | 92.590 | 92.587 | 92.362 |
| 670 | 94.693 | 93.390 | 92.622 | 92.650 | 92.421 |
| 680 | 94.699 | 93.436 | 92.676 | 92.712 | 92.475 |
| 690 | 94.672 | 93.425 | 92.684 | 92.733 | 92.466 |
| 700 | 94.617 | 93.405 | 92.649 | 92.736 | 92.465 |
| 710 | 94.595 | 93.395 | 92.660 | 92.731 | 92.452 |
| 720 | 94.548 | 93.378 | 92.636 | 92.746 | 92.440 |
| 730 | 94.668 | 93.492 | 92.772 | 92.866 | 92.552 |
| 740 | 94.762 | 93.605 | 92.859 | 93.002 | 92.681 |
| 750 | 94.908 | 93.826 | 92.966 | 93.274 | 92.913 |

| | | | | | |
|---|---|---|---|---|---|
| *Reference | | | | | |

**TABLE 15**

| *Reflectance of Examples 3A - 3B and Example 3 Control* | | | |
|---|---|---|---|
| **Wavelength (nm)** | **Example 3A* (18.2%)** | **Example 3B* (2.2%)** | **Example 3* Control (0%)** |
| 360 | 36.197 | 44.088 | 46.48 |
| 370 | 50.591 | 60.687 | 64.103 |
| 380 | 64.071 | 72.756 | 76.585 |
| 390 | 74.915 | 79.579 | 83.110 |
| 400 | 81.752 | 83.286 | 86.580 |
| 410 | 85.343 | 85.385 | 88.712 |
| 420 | 87.416 | 86.731 | 90.188 |
| 430 | 88.773 | 87.571 | 91.196 |
| 440 | 89.879 | 88.266 | 92.054 |
| 450 | 90.812 | 88.865 | 92.785 |
| 460 | 91.622 | 89.346 | 93.400 |
| 470 | 92.349 | 89.828 | 93.968 |
| 480 | 92.938 | 90.225 | 94.449 |
| 490 | 93.319 | 90.504 | 94.758 |
| 500 | 93.750 | 90.849 | 95.098 |
| 510 | 94.034 | 91.084 | 95.292 |
| 520 | 94.227 | 91.266 | 95.440 |
| 530 | 94.405 | 91.448 | 95.559 |
| 540 | 94.611 | 91.649 | 95.692 |
| 550 | 94.711 | 91.769 | 95.751 |
| 560 | 94.821 | 91.896 | 95.812 |
| 570 | 94.902 | 92.013 | 95.858 |
| 580 | 95.004 | 92.152 | 95.926 |
| 590 | 95.089 | 92.290 | 95.977 |
| 600 | 95.122 | 92.398 | 96.008 |
| 610 | 95.079 | 92.439 | 95.946 |
| 620 | 95.159 | 92.594 | 96.019 |
| 630 | 95.202 | 92.705 | 96.030 |
| 640 | 95.297 | 92.893 | 96.123 |
| 650 | 95.328 | 92.996 | 96.138 |
| 660 | 95.407 | 93.133 | 96.200 |
| 670 | 95.367 | 93.184 | 96.163 |
| 680 | 95.386 | 93.269 | 96.162 |
| 690 | 95.373 | 93.306 | 96.100 |
| 700 | 95.296 | 93.311 | 95.989 |
| 710 | 95.290 | 93.359 | 95.955 |
| 720 | 95.253 | 93.386 | 95.895 |
| 730 | 95.366 | 93.561 | 96.014 |
| 740 | 95.480 | 93.745 | 96.118 |
| 750 | 95.638 | 93.981 | 96.270 |

| | | | |
|---|---|---|---|
| *Reference | | | |

**TABLE 16**

| *Reflectance of Examples 4A-4D and Example 4 Control* | | | | | |
|---|---|---|---|---|---|
| **Wavelength (nm)** | **Example 4A* (16.0%)** | **Example 4B* (9.9%)** | **Example 4C* (5.0%)** | **Example 4D* (1.2%)** | **Example 4 Control (0%)** |
| 360 | 26.958 | 24.228 | 18.745 | 22.122 | 22.65 |
| 370 | 27.573 | 24.349 | 19.639 | 22.244 | 22.639 |
| 380 | 27.624 | 23.949 | 20.667 | 21.909 | 22.143 |
| 390 | 31.171 | 27.224 | 24.934 | 25.104 | 25.104 |
| 400 | 37.445 | 33.583 | 31.988 | 31.377 | 30.986 |
| 410 | 55.503 | 52.738 | 51.249 | 50.262 | 49.482 |
| 420 | 88.135 | 88.755 | 85.541 | 85.464 | 86.578 |
| 430 | 109.019 | 112.592 | 107.557 | 108.292 | 112.049 |
| 440 | 117.182 | 121.542 | 115.378 | 116.723 | 121.699 |
| 450 | 110.478 | 113.787 | 108.641 | 109.135 | 113.782 |
| 460 | 102.865 | 105.017 | 101.231 | 100.680 | 104.566 |
| 470 | 100.722 | 102.352 | 99.020 | 98.164 | 101.770 |
| 480 | 98.470 | 99.602 | 96.763 | 95.621 | 98.900 |
| 490 | 94.931 | 95.490 | 93.347 | 91.809 | 94.557 |
| 500 | 93.863 | 94.154 | 92.342 | 90.712 | 93.110 |
| 510 | 92.692 | 92.754 | 91.226 | 89.585 | 91.643 |
| 520 | 91.088 | 90.927 | 89.723 | 88.054 | 89.733 |
| 530 | 89.835 | 89.511 | 88.534 | 86.897 | 88.227 |
| 540 | 89.235 | 88.793 | 87.980 | 86.426 | 87.476 |
| 550 | 88.758 | 88.232 | 87.516 | 86.075 | 86.870 |
| 560 | 88.265 | 87.648 | 87.040 | 85.688 | 86.267 |
| 570 | 87.857 | 87.165 | 86.628 | 85.348 | 85.754 |
| 580 | 87.767 | 87.032 | 86.511 | 85.331 | 85.601 |
| 590 | 87.962 | 87.203 | 86.681 | 85.605 | 85.774 |
| 600 | 88.186 | 87.436 | 86.902 | 85.935 | 86.004 |
| 610 | 88.247 | 87.489 | 86.969 | 86.055 | 86.059 |
| 620 | 88.345 | 87.564 | 87.050 | 86.192 | 86.126 |
| 630 | 88.485 | 87.713 | 87.196 | 86.390 | 86.259 |
| 640 | 88.856 | 88.101 | 87.583 | 86.820 | 86.618 |
| 650 | 89.229 | 88.493 | 87.982 | 87.267 | 86.996 |
| 660 | 89.630 | 88.912 | 88.406 | 87.722 | 87.415 |
| 670 | 89.861 | 89.117 | 88.657 | 87.981 | 87.632 |
| 680 | 90.007 | 89.242 | 88.819 | 88.141 | 87.754 |
| 690 | 90.065 | 89.310 | 88.887 | 88.236 | 87.809 |
| 700 | 90.066 | 89.327 | 88.896 | 88.277 | 87.821 |
| 710 | 90.087 | 89.341 | 88.915 | 88.305 | 87.836 |
| 720 | 90.076 | 89.337 | 88.911 | 88.314 | 87.827 |
| 730 | 90.184 | 89.429 | 88.997 | 88.424 | 87.924 |
| 740 | 90.261 | 89.513 | 89.054 | 88.501 | 87.968 |
| 750 | 90.341 | 89.682 | 89.110 | 88.600 | 87.987 |

| | | | | | |
|---|---|---|---|---|---|
| *Reference | | | | | |

**TABLE 17A**

| ***Reflectance of Example 5A and Example 5A Control*** | | | | |
|---|---|---|---|---|
| | **(Measured through Nylon Side)** | | **(Measured through Polyester side)** | |
| **Wavelength (nm)** | **Example 5A** | **Example 5A Control** | **Example 5A** | **Example 5A Control** |
| | **(14.4%)** | **(0%)** | **(14.4%)** | **(0%)** |
| 360 | 10.80 | 7.43 | 21.37 | 18.89 |
| 370 | 9.041 | 6.082 | 24.638 | 22.082 |
| 380 | 8.722 | 5.753 | 26.246 | 23.578 |
| 390 | 11.341 | 7.747 | 28.274 | 25.351 |
| 400 | 24.935 | 18.686 | 36.268 | 32.439 |
| 410 | 57.559 | 47.297 | 56.612 | 50.811 |
| 420 | 95.044 | 86.489 | 83.974 | 77.617 |
| 430 | 119.201 | 116.550 | 104.485 | 101.018 |
| 440 | 122.047 | 122.348 | 108.518 | 107.630 |
| 450 | 115.143 | 115.889 | 104.350 | 104.276 |
| 460 | 106.568 | 106.917 | 98.606 | 98.605 |
| 470 | 100.533 | 100.502 | 94.571 | 94.513 |
| 480 | 97.432 | 97.198 | 92.576 | 92.554 |
| 490 | 94.413 | 93.919 | 90.606 | 90.494 |
| 500 | 92.877 | 92.215 | 89.689 | 89.511 |
| 510 | 91.403 | 90.511 | 88.843 | 88.550 |
| 520 | 90.295 | 89.189 | 88.263 | 87.839 |
| 530 | 89.560 | 88.274 | 87.296 | 87.372 |
| 540 | 89.197 | 87.774 | 87.793 | 87.111 |
| 550 | 88.955 | 87.412 | 87.679 | 86.899 |
| 560 | 88.820 | 87.179 | 87.629 | 86.774 |
| 570 | 88.827 | 87.140 | 87.725 | 86.805 |
| 580 | 88.860 | 87.123 | 87.803 | 86.836 |
| 590 | 88.935 | 87.179 | 87.915 | 86.929 |
| 600 | 89.114 | 87.385 | 88.138 | 87.175 |
| 610 | 89.251 | 87.585 | 88.306 | 87.416 |
| 620 | 89.504 | 87.940 | 88.598 | 87.792 |
| 630 | 89.810 | 88.340 | 88.939 | 88.222 |
| 640 | 90.234 | 88.816 | 89.376 | 88.734 |
| 650 | 90.551 | 89.181 | 89.746 | 89.121 |
| 660 | 90.904 | 89.584 | 90.126 | 89.561 |
| 670 | 91.219 | 89.986 | 90.478 | 89.975 |
| 680 | 91.483 | 90.270 | 90.764 | 90.285 |
| 690 | 91.750 | 90.559 | 91.050 | 90.588 |
| 700 | 91.886 | 90.729 | 91.216 | 90.777 |
| 710 | 91.971 | 90.809 | 91.310 | 90.884 |
| 720 | 92.014 | 90.846 | 91.373 | 90.940 |
| 730 | 92.025 | 90.852 | 91.418 | 90.956 |
| 740 | 91.960 | 90.767 | 91.374 | 90.886 |
| 750 | 91.847 | 90.625 | 91.276 | 90.739 |

**TABLE17B**

| *Reflectance of Example 5B and Example 5B Control* | | | | |
|---|---|---|---|---|
| | **(Measured through Nylon side)** | | **(Measured through Polyester side)** | |
| **Wavelength (nm)** | **Example 5B** | **Example 5B Control** | **Example 5B** | **Example 5B Control** |
| | **(20.1%)** | **(0%)** | **(20.1%)** | **(0%)** |
| 360 | 48.20 | 37.37 | 39.09 | 33.06 |
| 370 | 59.510 | 49.121 | 53.704 | 46.976 |
| 380 | 66.743 | 56.887 | 62.759 | 55.918 |
| 390 | 71.565 | 61.994 | 68.191 | 61.273 |
| 400 | 75.052 | 65.964 | 72.033 | 65.221 |
| 410 | 77.447 | 68.948 | 74.745 | 68.209 |
| 420 | 79.281 | 71.376 | 76.795 | 70.602 |
| 430 | 80.807 | 73.465 | 78.740 | 72.662 |
| 440 | 82.115 | 75.283 | 79.959 | 74.508 |
| 450 | 83.300 | 76.950 | 81.345 | 76.228 |
| 460 | 84.433 | 78.532 | 82.686 | 77.895 |
| 470 | 85.412 | 79.930 | 83.859 | 79.391 |
| 480 | 86.268 | 81.181 | 84.899 | 80.736 |
| 490 | 86.916 | 82.140 | 85.701 | 81.771 |
| 500 | 87.545 | 83.042 | 86.457 | 82.737 |
| 510 | 87.959 | 83.664 | 86.976 | 83.407 |
| 520 | 88.266 | 84.137 | 87.361 | 83.924 |
| 530 | 88.566 | 84.543 | 87.733 | 84.370 |
| 540 | 88.861 | 84.923 | 88.095 | 84.779 |
| 550 | 89.049 | 85.181 | 88.337 | 85.073 |
| 560 | 89.215 | 85.394 | 88.552 | 85.319 |
| 570 | 89.435 | 85.655 | 88.817 | 85.603 |
| 580 | 89.615 | 85.853 | 89.051 | 85.839 |
| 590 | 89.795 | 86.066 | 89.272 | 86.086 |
| 600 | 90.027 | 86.393 | 89.543 | 86.421 |
| 610 | 90.200 | 86.641 | 89.749 | 86.710 |
| 620 | 90.468 | 86.998 | 90.045 | 87.110 |
| 630 | 90.777 | 87.386 | 90.402 | 87.542 |
| 640 | 91.157 | 87.825 | 90.819 | 88.034 |
| 650 | 91.441 | 88.149 | 91.137 | 88.434 |
| 660 | 91.746 | 88.493 | 91.474 | 88.817 |
| 670 | 92.024 | 88.839 | 91.784 | 89.156 |
| 680 | 92.246 | 89.091 | 92.033 | 89.445 |
| 690 | 92.544 | 89.421 | 92.351 | 89.822 |
| 700 | 92.732 | 89.669 | 92.562 | 90.100 |
| 710 | 92.854 | 89.841 | 92.703 | 90.300 |
| 720 | 92.939 | 89.982 | 92.808 | 90.463 |
| 730 | 92.997 | 90.074 | 92.876 | 90.575 |
| 740 | 92.981 | 90.070 | 92.858 | 90.597 |
| 750 | 92.922 | 89.987 | 92.796 | 90.546 |

**TABLE 18**

| *Reflectance of Comparative Examples A-E* | | | | | |
|---|---|---|---|---|---|
| **Wavelength (nm)** | **Comparative Example A** | **Comparative Example B** | **Comparative Example C** | **Comparative Example D** | **Comparative Example E** |
| 360 | 14.720 | 19.650 | 20.716 | 10.151 | 33.155 |
| 370 | 23.794 | 21.652 | 23.292 | 10.477 | 34.267 |
| 380 | 29.113 | 22.815 | 25.320 | 10.525 | 34.831 |
| 390 | 30.961 | 23.679 | 27.120 | 10.464 | 35.830 |
| 400 | 31.480 | 24.275 | 28.441 | 10.372 | 37.109 |
| 410 | 31.630 | 24.835 | 29.436 | 10.257 | 38.212 |
| 420 | 31.694 | 25.182 | 29.968 | 10.105 | 38.758 |
| 430 | 31.664 | 24.964 | 29.706 | 9.919 | 38.727 |
| 440 | 31.600 | 24.585 | 29.115 | 9.745 | 38.287 |
| 450 | 31.516 | 23.989 | 28.151 | 9.612 | 37.669 |
| 460 | 31.439 | 23.446 | 27.120 | 9.552 | 37.066 |
| 470 | 31.342 | 23.056 | 26.191 | 9.549 | 36.597 |
| 480 | 31.253 | 22.736 | 25.302 | 9.626 | 36.237 |
| 490 | 31.110 | 22.432 | 24.416 | 9.757 | 35.878 |
| 500 | 31.040 | 22.283 | 23.712 | 9.976 | 35.583 |
| 510 | 30.895 | 22.123 | 23.033 | 10.203 | 35.230 |
| 520 | 30.769 | 22.025 | 22.446 | 10.491 | 34.932 |
| 530 | 30.601 | 21.937 | 21.919 | 10.780 | 34.645 |
| 540 | 30.476 | 21.935 | 21.533 | 11.130 | 34.485 |
| 550 | 30.302 | 21.938 | 21.212 | 11.472 | 34.381 |
| 560 | 30.153 | 21.986 | 20.989 | 11.844 | 34.404 |
| 570 | 29.999 | 22.072 | 20.846 | 12.229 | 34.551 |
| 580 | 29.871 | 22.213 | 20.798 | 12.644 | 34.852 |
| 590 | 29.716 | 22.365 | 20.801 | 13.048 | 35.224 |
| 600 | 29.580 | 22.580 | 20.877 | 13.511 | 35.754 |
| 610 | 29.472 | 22.822 | 20.988 | 13.967 | 36.322 |
| 620 | 29.364 | 23.094 | 21.122 | 14.420 | 36.953 |
| 630 | 29.223 | 23.361 | 21.249 | 14.853 | 37.575 |
| 640 | 29.102 | 23.684 | 21.392 | 15.291 | 38.239 |
| 650 | 28.945 | 24.012 | 21.520 | 15.709 | 38.868 |
| 660 | 28.810 | 24.390 | 21.663 | 16.139 | 39.537 |
| 670 | 28.659 | 24.800 | 21.818 | 16.565 | 40.202 |
| 680 | 28.518 | 25.262 | 21.996 | 16.995 | 40.899 |
| 690 | 28.515 | 25.884 | 22.323 | 17.538 | 41.734 |
| 700 | 28.527 | 26.602 | 22.722 | 18.110 | 42.611 |
| 710 | 28.468 | 27.286 | 23.094 | 18.611 | 43.427 |
| 720 | 28.386 | 27.986 | 23.488 | 19.086 | 44.216 |
| 730 | 28.197 | 28.651 | 23.866 | 19.462 | 44.937 |
| 740 | 27.907 | 29.281 | 24.234 | 19.758 | 45.574 |
| 750 | 27.323 | 29.804 | 24.524 | 19.892 | 46.068 |

The reflectance data from Examples with the highest silver loadings (that is, **Examples 2A, 3A, 4A,** and **5B**), along with the reflectance data from the Comparative Examples, is plotted in line graph in **FIGURE 1****.**

From the data, one observes that the present **Examples** exhibit significantly higher reflectance across the visible spectrum than do any of the **Comparative Examples.** These values are indicative of the white (highly reflective) nature of the present treated articles.

A review of **FIGURE 1** indicates that **Examples 2A, 3A, 4A,** and **5B** are grouped fairly closely together on a line graph, all positioned around the plot of the reflectance data for a white reference tile, where wavelength (in nm) is the x axis and reflectance (%) is the y axis. This indicates that processing conditions have been established to ensure a treated article with a white surface, regardless of the amount of silver-based antimicrobial applied. Even with significant amounts of silver antimicrobial, such as the 20.7% of **Example 2A** and the 20.1% of **Example 5B,** the color of the treated article is not adversely affected.

The peak in reflectance values that is observed at wavelengths of between 400 nm and 500 nm in **Example 4A** is due to the presence of an optical brightener. Such brighteners are known to further enhance the reflectivity of an article, but are not required to produce a white color in the present devices.

### TEST 5: Whiteness / Yellowness Evaluation

Whiteness is measured using the Stensby Index for Whiteness. Higher values indicate a fabric with greater whiteness.

A correlating measure is ASTM E313-73 (D1925), which measures the yellowness of a sample. Higher values indicate a sample with more yellowness; negative values indicate a sample with less yellowness.

The Example fabrics (2-5) were measured, as described, with comparison being made to a white reference tile. The white ceramic tile was calibrated and traceable to the

National Institute of Standards & Technology (NIST) and is referenced by the (NIST) report, standard reference material 2020c Serial number 27073, 844/259504-98R dated May 14, 1999. The Ceramic Standards are calibrated to the GRETAG MACBETH® Virtual database (STF19 Sphere). Calibration values displayed are CIELAB 1976, Illuminant D65, and 10° Observer at a controlled temperature of between 71°F and 73 °F.

The tests were run using GretagMacbeth Color Eye 7000A spectrophotometer, including a xenon flash light source, with results being provided in **TABLES 19A** and **19B.**

**TABLE 19A**

| *Whiteness* / *Yellowness Measurements: Examples 2-4* | | |
|---|---|---|
| **Sample Identification** | **Whiteness (Stensby Index)** | **Yellowness (ASTM E313-73 (D1925))** |
| White Reference Tile | 89.317 | 9.210 |
| Example 2A * | 80.622 | 14.233 |
| Example 2B * | 77.545 | 15.456 |
| Example 2C * | 82.669 | 13.260 |
| Example 2D * | 75.450 | 16.479 |
| Example 2 Control | 81.702 | 13.735 |
| Example 3A * | 87.117 | 11.713 |
| Example 3B * | 88.961 | 11.040 |
| Example 3 Control | 89.985 | 10.636 |
| Example 4A * | 128.443 | -7.284 |
| Example 4B * | 135.182 | -10.349 |
| Example 4C * | 126.565 | -6.920 |
| Example 4 D * | 131.201 | -8.165 |
| Example 4 Control | 136.404 | -11.474 |

| | | |
|---|---|---|
| *Reference | | |

**TABLE 19B**

| *Whiteness* / *Yellowness Measurements: Examples 5A and 5B* | | | |
|---|---|---|---|
| **Sample Identification** | **Optical Brightener** | **Whiteness (Stensby Index)** | **Yellowness (ASTM E313-73 (D1925))** |
| White Reference Tile. | n/a | 85.799 | 11.812 |
| Example 5A (nylon side) | Yes | 142.318 | -9.885 |
| Example 5A Control (nylon side) | Yes | 141.381 | -10.773 |
| Example 5A (polyester side) | Yes | 121.151 | -2.038 |
| Example 5A Control (polyester side) | Yes | 118.114 | -1.807 |
| Example 5B (nylon side) | No | 80.489 | 14.295 |
| Example 5B Control (nylon side) | No | 71.776 | 17.427 |
| Example 5B (polyester side) | No | 77.268 | 15.762 |
| Example 5B Control (polyester side) | No | 70.546 | 18.172 |

The data in **TABLES 19A** and **19B** indicates that, generally, the silver loading does not significantly affect the color of the device. In other words, a manufacturer can apply compositions containing various amounts of silver (and specifically, ALPHASAN® antimicrobial) to a substrate and still maintain a white color in the treated substrate. Heretofore, with other known silver compounds, it has been impossible to obtain a white treated substrate with the effective amounts of silver necessary for wound treatment and infection prevention.

### TEST 6: Color Evaluafion: Lightness / Darkness, Yellow / Blue, Red / Green

Often, the surface color of an article is quantified using a series of measurements (L*, a*, and b*) generated by measuring the samples using a spectrophotometer. The equipment used for this test was a GretagMacbeth Color Eye 7000A spectrophotometer. The software program used was "Color imatch." "L" is a measure of the amount of white or black in a sample; higher "L" values indicate a lighter colored sample. "A" is a measure of the amount of red or green in a sample, while "B" is a measure of the amount of blue or yellow in a sample.

Other measures made using the same testing equipment include C* and h°. C*, chroma, is a measure of the color saturation of the article. h°, hue, is a measure of the shade of the article.

**TABLES 20A and 20B show** a comparison of various samples, as tested by a GretagMacbeth Color Eye 7000A spectrophotometer using the white reference tile described in Test 4 as a standard. It is important to note that the values obtained are dependent upon the type of simulated light source used (e.g., incandescent, fluorescent, etc.) and the angle of observation. In these tests, a D65-10 setting was used (which represents daylight conditions), 6500 °K is the correlated color temperature, and 10° is the angle of observation.

**TABLE 20A**

| *Color Measurements of Examples 2 - 4 and Comparative Examples* | | | | | | |
|---|---|---|---|---|---|---|
| **Sample ID (% ALPHASAN® Content)** | **Sample Color** | **L*** | **C*** | **h°** | **a*** | **b*** |
| White reference tile | White | 95.587 | 1.235 | 114.481 | -0.512 | 1.124 |
| Example 2A* (20.7%) | White | 97.332 | 4.382 | 102.328 | -0.936 | 4.281 |
| Example 2B*(8.2%) | White | 96.611 | 5.091 | 100.910 | -0.964 | 4.999 |
| Example 2C*(4.2%) | White | 96.433 | 3.599 | 100.175 | -0.636 | 3.542 |
| Example 2D*(1.1%) | White | 96.079 | 5.636 | 99.216 | -0.903 | 5.563 |
| Ex. 2 Control (0%) | White | 96.255 | 3.860 | 99.244 | -0.620 | 3.810 |
| Example 3A* (18.2%) | White | 97.808 | 2.721 | 102.936 | -0.609 | 2.652 |
| Example 3B*(2.2%) | White | 96.668 | 2.046 | 94.481 | -0.160 | 2.040 |
| Ex. 3 Control (0%) | White | 98.248 | 2.051 | 104.613 | -0.517 | 1.985 |
| Example 4A* (16.0%) | White | 96.174 | 8.887 | 280.515 | 1.622 | -8738 |
| Example 4B*(9.9%) | White | 96.060 | 10.626 | 280.689 | 1.971 | -10.441 |
| Example 4C*(5.0%) | White | 95.618 | 8.621 | 280.348 | 1.549 | -8.481 |
| Example 4D*(1.2%) | White | 95.082 | 9.658 | 283.814 | 2.306 | -9.379 |
| Ex. 4 Control (0%) | White | 95.534 | 11.162 | 280.374 | 2.010 | -10.980 |
| Comparative Ex. A | Black interior; white outer layers | 61.900 | 1.898 | 245.779 | -0.779 | -1.731 |
| Comparative Ex. B | Dark bluish-gray metallic | 54.396 | 3.595 | 310.716 | 2.345 | -2725 |
| Comparative Ex. C | Dark bluish-grays metallic | 54.025 | 9.870 | 281.791 | 2.017 | -9.662 |
| Comparative Ex. D | Dark gray | 40.804 | 7.903 | 48.557 | 5.231 | 5.925 |
| Comparative Ex. E | Light blue-gray | 65.994 | 3.927 | 313.587 | 2.708 | -2.845 |

| | | | | | | |
|---|---|---|---|---|---|---|
| *Reference | | | | | | |

**TABLE 20B**

| *Color Measurements of Examples 5 and 6* | | | | | | |
|---|---|---|---|---|---|---|
| **Sample ID (Side on which measurement was made)** | **% ALPHASAN® Content** | **L*** | **C*** | **h°** | **a*** | **b*** |
| White reference tile | n/a | 95.513 | 2.523 | 95.953 | -0.262 | 2.509 |
| Example 5A (nylon side) | 14.4% | 96.332 | 11.471 | 288.519 | 3.643 | -10.877 |
| Example 5A Control (nylon side) | 0% | 95.779 | 11.627 | 286.509 | 3.304 | -11.147 |
| Example 5A (polyester side) | 14.4% | 95.518 | 6.618 | 290.143 | 2.279 | -6.214 |
| Example 5A Control (polyester side) | 0% | 95.223 | 6.142 | 286.884 | 1.784 | -5.878 |
| Example 5B (nylon side) | 20.1% | 95.489 | 4.081 | 96.754 | -0.480 | 4.053 |
| Example 5B Control (nylon side) | 0% | 93.771 | 6.037 | 97.857 | -0.825 | 5.981 |
| Example 5B (polyester side) | 20.1% | 95.162 | 4.969 | 96.393 | -0.553 | 4.938 |
| Example 5B Control (polyester side) | 0% | 93.716 | 6.460 | 97.150 | -0.804 | 6.410 |
| Example 6 * (nylon side) | n/a | 67.814 | 11.041 | 291.904 | 4.119 | -10.244 |
| Example 6 * (polyester side) | n/a | 68.382 | 4.214 | 291.399 | 1.537 | -3.923 |

| | | | | | | |
|---|---|---|---|---|---|---|
| *Reference Looking at the L* values in **TABLES 20A** and **20B** for **Examples 2 - 6,** it is apparent that there is a slight reduction in L* values as the amount of silver-containing antimicrobial agent (ALPHASAN®) is decreased. As noted, **Examples 4A - 4D** and **5A** contain an optical brightening agent, which causes the Examples to emit blue light and which results in the higher h° values shown in **TABLES 20A** and **20B.** | | | | | | |

**TABLE 20A** shows that the Comparative Examples each have an L* value of less than about 66, indicating the presence of darker shades of color. The present wound care devices have an L* value of at least about 68 (for instance, when nanoparticle silver is used) and at least about 95 (for instance, when silver ion exchange compounds are used).

One contemplated benefit of having an antimicrobial wound care device whose color has not been altered by the addition of a silver antimicrobial agent is that the wound care device can be dyed a number of different colors. For example, the wound care devices could be dyed to represent different end uses or levels of antimicrobial agent. Representative colored wound care substrates were prepared as described above. **Examples 7A - 7F** and the corresponding Control samples were measured using the color evaluation techniques and equipment described previously. The results are shown in **TABLES 21A** and **21B.**

**TABLE 21A**

| *Color Evaluation: Examples 7A-7F and Corresponding Control Samples (measured through the nylon side of the fabric with D-65 light source)* | | | | | | | |
|---|---|---|---|---|---|---|---|
| **Sample ID** | **Sample Color** | **L*** | **a*** | **b*** | **C*** | **h°** | **DE CMC** |
| Reference tile | white | 95.518 | -0.278 | 2.498 | 2.513 | 96.358 | n/a |
| Example 7A | blue | 75.609 | -11.036 | -13.713 | 17.602 | 231.173 | 26.006 |
| Control 7A | blue | 72.076 | -10.778 | -14.562 | 18.116 | 233.493 | 27.052 |
| Example 7B | blue | 82.515 | -8.789 | -9.976 | 13.295 | 228.618 | 20.077 |
| Control 7B | blue | 77.970 | -9.585 | -11.618 | 15.061 | 230.475 | 22.682 |
| Example 7C | green | 84.542 | -11.911 | 21.160 | 24.282 | 119.375 | 28.010 |
| Control 7C | green | 81.022 | -13.322 | 23.755 | 27.236 | 119.285 | 31.864 |
| Example 7D | green | 88.135 | -9.642 | 18.373 | 20.750 | 117.690 | 23.402 |
| Control 7D | green | 85.300 | -10.861 | 20.531 | 23.227 | 117.880 | 26.620 |
| Example 7E | purple | 74.010 | 7.616 | -12.837 | 14.541 | 301.587 | 23.055 |
| Control 7E | purple | 68.487 | 7.725 | -14.171 | 16.139 | 298.595 | 25.660 |
| Example 7F | purple | 80.969 | 5.585 | -8.701 | 10.340 | 302.697 | 17.240 |
| Control 7F | purple | 75.808 | 6.527 | -10.181 | 12.094 | 302.666 | 19.907 |

**TABLE 21B**

| *Color Evaluation: Examples 7A - 7F and Corresponding Control Samples (measured through the polyester side of the fabric with D-65 light source)* | | | | | | | |
|---|---|---|---|---|---|---|---|
| **Sample ID** | **Sample Color** | **L*** | **a*** | **b*** | **C*** | **h°** | **DE CMC** |
| Reference tile | white | 95.518 | -0.278 | 2.498 | 2.513 | 96.358 | n/a |
| Example 7A | blue | 82.818 | -5.967 | -7.422 | 9.523 | 231.201 | 15.557 |
| Control 7A | blue | 78.653 | -5.948 | -8.324 | 10.231 | 234.454 | 16.953 |
| Example 7B | blue | 85.706 | -5.434 | -5.993 | 8.090 | 227.803 | 13.444 |
| Control 7B | blue | 81.367 | -6.069 | -7.245 | 9.451 | 230.050 | 15.578 |
| Example 7C | green | 87.336 | -7.057 | 12.500 | 14.354 | 119.447 | 15.523 |
| Control 7C | green | 84.051 | -8.241 | 13.740 | 16.008 | 120.872 | 17.827 |
| Example 7D | green | 89.884 | -5.869 | 10.917 | 12.395 | 118.262 | 12.919 |
| Control 7D | green | 87.047 | -7.015 | 12.529 | 14.359 | 119.247 | 15.542 |
| Example 7E | purple | 82.736 | 3.165 | -6.167 | 6.932 | 297.169 | 13.036 |
| Control 7E | purple | 77.424 | 3.566 | -7.519 | 8.321 | 295.375 | 15.383 |
| Example 7F | purple | 85.972 | 3.027 | -4.732 | 5.617 | 302.611 | 11.006 |
| Control 7F | purple | 80.853 | 3.706 | -6.258 | 7.272 | 300.633 | 13.615 |

The results shown in **TABLES 21A** and **21B** indicate that the application of the antimicrobial finish described herein does not adversely alter the overall color of the wound care substrate (that is, the DE CMC values are substantially the same between each Example and its Control).

Further, the Example fabrics exhibited an L* value of at least 74.0, a relatively high level of lightness, which is desirable for wound fluid monitoring purposes.

### TEST 7: Comparison of Magnitude of Color Difference between Ample and Reference Tile

Yet another measurement of the relative color of the samples is DE CMC. DE CMC is a measure of the overall color difference for all uniform color spaces, where DE CMC represents the magnitude of difference between a color and a reference (in this case, a pure white standard). The higher the DE CMC value, the more pronounced the difference in color. Said another way, smaller DE CMC values represent colors that are closer to white.

The GretagMacbeth Color Eye 7000A Spectrophotometer calculates DE CMC values based on wavelength and reflectance data for each sample. Examples 2A - 2D, 3A - 3B, 4A - 4D, and 5A - 5B were compared to the Comparative Examples. The results are shown in **TABLES 22A** and **22B.**

**TABLE 22A**

| *DE CMC: Comparison of Magnitude of Color Difference* | | |
|---|---|---|
| **Sample Identification (% active antimicrobial)** | **L* value (Lightness of sample)** | **DE CMC** |
| Example 2A*(20.7%) | 97.332 | 4.493 |
| Example 2B*(8.2%) | 96.611 | 5.465 |
| Example 20*(4.2%) | 96.433 | 3.398 |
| Example 2D*(1.1%) | 96.079 | 6.231 |
| Example 2 Control (0%) | 96.255 | 3.765 |
| Example 39(18.2%) | 97.808 | 2.273 |
| Example 3B*(2.2%) | 96.668 | 1.425 |
| Example 3 Control (0%) | 98.248 | 1.511 |
| Example 4A*(16.0%) | 96.174 | 14.166 |
| Example 4B*(9.9%) | 96.060 | 16.596 |
| Example 4C*(5.0%) | 95.618 | 13.791 |
| Example 4D*(1.2%) | 95.082 | 15.262 |
| Example 4 Control (0%) | 95.534 | 17.343 |
| Example 5A (14.4%; nylon side) | 96.332 | 18.171 |
| Example 5A Control (0%; nylon side) | 95.779 | 18.387 |
| Example 5A*(14.4%; polyester side) | 95.518 | 11.974 |
| Example 5A Control (0%;polyester side) | 95.223 | 11.395 |
| Example 5B (20.1%; nylon side) | 95.489 | 1.964 |
| Example 5B Control (0%; nylon side) | 93.771 | 4.472 |
| Example 5B (20.1%; polyester side) | 95.162 | 3.084 |
| Example 5B Control (0%;polyester side) | 93.716 | 5.000 |

| | | |
|---|---|---|
| *Reference | | |

The data shown in **TABLES 21A, 21B,** and **22A** indicates that the application of the antimicrobial finish described herein does not adversely alter the overall color of the wound care substrate (that is, the DE CMC values of the Example fabrics are approximately equal to or less than the DE CMC values of the respective Control fabrics). This is true whether the sample is dyed (as in **TABLES 21A** and **21B**) or whether the sample is left undyed (i.e., white) (as in **TABLE 22A**).

**TABLE 22B**

| *DE CMC: Comparison of Magnitude of Color Difference* | | |
|---|---|---|
| **Sample Identification (% active antimicrobial)** | **L* value (Lightness of sample)** | **DE CMC** |
| Comparative Example A | 61.900 | 12.244 |
| Comparative Example B | 54.396 | 15.661 |
| Comparative Example C | 54.025 | 21.094 |
| Comparative Example D | 40.804 | 21.519 |
| Comparative Example E | 65.994 | 12.443 |

The results shown in **TABLES 22A** and **22B** indicate that, whereas the experimental Examples without optical brighteners exhibited DE CMC value no higher than 6.2, all of the Comparative Examples exhibited a DE CMC value higher than 12. This finding indicates that the Examples prepared in accordance with the teachings herein exhibited less difference from the white standard than did the Comparative Examples.

### TEST 8: Color Stability Testing

It is desirable that the color of the antimicrobial wound care device does not change significantly with the passage of time. With traditional silver-based antimicrobial devices, exposure to light causes a color change in the form of browning or graying. The present device overcomes this shortcoming.

To evaluate the color durability of the present device, the yellowness of treated Example 2E fabric and untreated Example 2 Control fabric were measured periodically over a 26-day period, using the test method described previously. Both fabrics were subjected to cool-white fluorescent lighting for 24-hour continuous exposure. Five different areas of each sample were evaluated and averaged. The data is reported below in **TABLE 23.**

**TABLE 23**

| *Color Durability Testing using Yellowness Index Measure* | | |
|---|---|---|
| **Days of Exposure** | **Control Fabric** | **Example 2E *** |
| 0 | 13.478 | 13.564 |
| 1 | 13.728 | 13.716 |
| 2 | 13.547 | 13.724 |
| 3 | 13.784 | 13.706 |
| 4 | 13.892 | 13.700 |
| 7 | 14.020 | 13.799 |
| 8 | 13.987 | 13.821 |
| 11 | 14.113 | 13.844 |
| 14 | 14.110 | 13.715 |
| 16 | 14.201 | 13.667 |
| 18 | 14.687 | 14.128 |
| 22 | 14.190 | 13.664 |
| 23 | 14.188 | 13.641 |
| 24 | 14.145 | 13.573 |
| 25 | 14.204 | 13.757 |

| | | |
|---|---|---|
| *Reference | | |

The results show that the **Example 2E** performed comparably with the untreated fabric in maintaining its original color over time. There were no visible signs of browning or graying, as are common with silver-containing articles that are exposed to the environment. Rather, **Example 2E** maintained its white appearance throughout the evaluation period.

### TEST 9 : Fluid Transpoort Test

The purpose of this test is to measure the amount of fluid that is transported from the wound contact side of the wound care device (Side A) to the non-wound contact side of the device (Side B). The test also attempts to measure the amount of fluid pushed back to the wound contact side of the device (Side A).

Simulated wound fluid ("SWF") was prepared by adding 16.60 g NaCl and 0.56 g CaCl₂ to a 2L volumetric flask. The flask was then filled to volume (2000mL total) with deionized water. The flask was then capped and shaken until all of the salts were completely dissolved. The simulated wound fluid is comprised of 0.142M (142 mM) NaCl (aq) and 0.0025M (2.5 mM) CaCl₂ (aq).

A test sample of a wound care device (5cm in diameter) was placed onto a polypropylene disc (5cm in diameter). Twenty drops of simulated wound fluid was added to Side A of the test sample using a dropper. The test sample was allowed to rest in a horizontal position for 2 minutes. The test sample was then sandwiched in a vertical position between two discs of filter paper (Whitman filter paper 3, diameter=110mm) using a clamp - Filter Paper A contacted Side A of the test sample and Filter Paper B contacted Side B of the test sample. The test sample was held in this position for 5 seconds. It was determined that the clamp exerts a pressure of 340 mm Hg.

Filter papers A and B had been weighed prior to the test. They were then weighed after the test and difference in weight was determined. This weight difference provides a calculation of the amount of SWF transferred from the wound care device to Filter Paper A and/or B. The SWF was added to the polyester side ("Side A") of the wound care device of the present invention. SWF was added to the wound contact side of competitive dressings, as directed by the product brochures.

Test results are shown in **TABLE 24.** The values are provided as "percent weight change." The percent weight change represents the weight of the fluid absorbed relative to the dry weight of the filter paper. It is calculated by subtracting the weight of the dry filter paper (grams) from the weight of the wet filter paper (grams) and dividing this difference by the weight of the dry filter paper. This value is then multiplied by 100. The average percent weight change for Side A and Side B for each Example and Comparative Example was also determined and is provided in **TABLE 24** as well. For instance, the percent weight change values for Side A and Side B for **Example 5B Control, Example 8A,** and **Example 8B** were averaged together. The results are shown as 4.8% and 24.2%, respectively. The standard deviation of the average value is also provided.

**TABLE 24**

| *Fluid Transport Properties of Inventive and Comparative Wound Care Devices (Provided* As *Percent Weight Change)* | | | | | | |
|---|---|---|---|---|---|---|
| **Sample** | **Side A Fluid** | **Side B fluid** | **Average Side A fluid** | **Standard Deviation** | **Average Side B fluid** | **Standard Deviation** |
| Example 5B Control- #1 (no antimicrobial agent) | 41% | 23.4% | 4.8% | 0.7% | 24.2% | 0.8% |
| Example 5B Control- #2 | 5.3 | 24.3 | | | | |
| Example 5B Control- #3 | 5.1 | 25.0 | | | | |
| Example 8A- #1 (ALPHASAN® RC 2000) | 8.2 | 17.8 | 9.8 | 1.8 | 18.4 | 0.5 |
| Example 8A- #2 | 9.3 | 18.8 | | | | 0 |
| Example 8A- #3 | 11.8 | 18.6 | | | | |
| Comp. Example C- #1 (ACTICOAT® 7) | 21.8 | 8.1 | 24.6 | 3.0 | 11.4 | 3.1 |
| Comp. Example C- #2 | 27.7 | 14.0 | | | | |
| Comp. Example C- #3 | 24.4 | 12.2 | | | | |
| Comp. Example D- #1 (SILVERLON®) | 5.5 | 6.0 | 6.2 | 0.6 | 2.2 | 3.3 |
| Comp. Example D- #2 | 6.8 | 0.1 | | | | |
| Comp. Example D- #3 | 6.3 | 0.7 | | | | |
| Comp. Example E- #1 (AQUACEL® Ag) | 2.6 | 2.0 | 2.3 | 0.2 | 1.8 | 0.2 |
| Comp. Example E- #2 | 2.2 | 1.5 | | | | |
| Comp. Example E- #3 | 2.1 | 1.8 | | | | |
| Comp. Example F- #1 (AQUACEL®) | 1.6 | 0.0 | 1.3 | 0.4 | 0.0 | 0.0 |
| Comp. Example F- #2 | 1.5 | 0.0 | | | | |
| Comp. Example F- #3 | 0.9 | 0.0 | | | | |

| **TABLE 24 (cont'd.)** | | | | | | |
|---|---|---|---|---|---|---|
| *Fluid Transport Properties of Inventive and Comparative Wound Care Devices (Provided As Percent Weight Change)* | | | | | | |
| **Sample** | **Side A Fluid** | **Side B fluid** | **Average Side A fluid** | **Standard Deviation** | **Average Side B fluid** | **Standard Deviation** |
| Comp. Example G- #1 (LYOFOAM®) | 23.2 | 12.7 | 27.3 | 3.9 | 10.2 | 2.5 |
| Comp. Example G- #2 | 30.9 | 10.0 | | | | |
| Comp. Example G- #3 | 27.9 | 7.8 | | | | |
| Comp. Example H- #1 (Foam Dressing) | 20.5 | 0.0 | 22.9 | 2.2 | 0.0 | 0.0 |
| Comp. Example H- #2 | 23.6 | 0.1 | | | | 0 |
| Comp. Example H- #3 | 24.7 | 0.1 | | | | |
| Comp. Example I- #1 (Woven Gauze Pad) | 18.1 | 17.8 | 19.4 | 2.2 | 16.5 | 1.4 |
| Comp. Example I- #2 | 18.2 | 16.6 | | | | |
| Comp. Example I- #3 | 21.9 | 15.1 | | | | |
| Comp. Example J- #1 (MIRASORB™ Gauze) | 31.0 | 1.6 | 29.4 | 2.1 | 2.3 | 1.0 |
| Comp. Example J- #2 | 27.0 | 1.8 | | | | |
| Comp. Example J- #3 | 30.1 | 3.5 | | | | |
| Comp. Example K- #1 (EVOLON®) | 14.0 | 11.6 | 13.0 | 1.4 | 12.2 | 2.0 |
| Comp. Example K- #2 | 13.6 | 10.6 | | | | |
| Comp. Example K- #3 | 11.4 | 14.5 | | | | |
| Comp. Example M- #1 (CONTREET® F) | 18.8 | 0.0 | 18.3 | 0.7 | 0.0 | 0.0 |
| Comp. Example M- #2 | 17.6 | 0.0 | | | | |
| Comp. Example M- #3 | 18.5 | 0.0 | | | | |

The results shown in **TABLE 24** demonstrate that the inventive wound care device provide significant movement of fluid away from the wound contact side of the device (Side A) to the reservoir side of the device (Side B). This directional fluid movement occurs even under pressure exerted from both sides of the wound care device. The results further show that the fluid movement is generally uni-directional in that the reservoir side (Side B) collects and holds most of the fluid and keeps it from flowing back to the wound contact side of the device (Side A).

These features are exhibited by the present device, whether or not the device also contains an antimicrobial agent.

It was also noted during this test that both of the AQUACEL® wound care devices fell apart. They were not able to hold together structurally after absorbing the SWF.

### TEST 10: Tensile Strength Test

Tensile strength (grab) of various wound care devices was determined using ASTM D 5034. The purpose of this test is to determine structural integrity of wet and dry wound care devices. The devices were wetted by dipping them in simulated wound fluid (same formulation as described previously). The test results are provided in **TABLE 25.** Measurements are shown in pounds of force (Ibf). Higher values indicate that more force was needed to tear the sample.

**TABLE 25**

| *Tensile Strength of Inventive and Comparative Wound Care Devices* | | | |
|---|---|---|---|
| **Sample ID** | **Sample Condition** | **Average of All Peaks (Ibf)** | **Average of All Peaks (Ibf)** |
| **Sample** | **Dry/Wet** | **Mean** | **Standard Deviation** |
| Example 5B Control (no antimicrobial agent) | Dry | 3.848 | 0.004 |
| Example 5B Control (no antimicrobial agent) | Wet | 5.106 | 0.216 |
| Example 8B (ALPHASAN® RC 2000) | Dry | 3.827 | 0.357 |
| Example 8B (ALPHASAN® RC 2000) | Wet | 4.561 | 0.300 |
| Comparative Example F (AQUACEL®) | Dry | 3.089 | 0.130 |
| Comparative Example F (AQUACEL®) | Wet | Sample fell apart when wetted. Test was not done. | Sample fell apart when wetted. Test was not done. |
| Comparative Example G (LYOFOAM®) | Dry | 1.163 | 0.012 |
| Comparative Example G (LYOFOAM®) | Wet | 1.013 | 0.014 |
| Comparative Example L (VERSALON™) | Dry | 0.563 | 0.030 |
| Comparative Example L (VERSALON™) | Wet | 0.595 | 0.133 |

### TEST 11 : Conductivity/Resistivity Test

The purpose of this test is to determine the conductivity and resistivity (R) of the inventive wound care device. The test was performed according to AATCC Test Method 76.

The test results are shown in **TABLE 26.** Each value is an average of four measurements. Log R also includes the standard deviation.

**TABLE 26**

| *Conductivity and Resistivity of Inventive Wound Care Device* | | | |
|---|---|---|---|
| **Sample ID** | **Conductivity (amperes)** | **Resistivity (ohms/sq.)** | **Log R (ohms/sq.)** |
| Example 5B Control (no antimicrobial) | 4.204E -11 | 2.147E+12 | 12.33 +/- 0.06 |
| Example 8A (ALPHASAN® RC 2000) | 3.958E-11 | 2.238E+12 | 12.34 +/- 0.07 |

The detection range of the testing equipment is in the range of 12 - 13 for the Log R value. Both **Example 5B Control** and **Example 8A** are below the detection limit of the testing equipment. Thus, the inventive wound care device is non-electrically conductive.

### TEST 12: Thickness Test

The purpose of this test was to measure the thickness of the inventive wound care device. The test was performed according to ASTM D 1777-96. The results are provided as an average with the standard deviation of ten measurements for each sample. The results are shown in **TABLE 27.**

**TABLE 27**

| *Thickness of Inventive Wound Care Device* | |
|---|---|
| **Sample ID** | **Average Thickness (mils)** |
| Example 5B Control (no antimicrobial) | 40.75 +/- 0.40 |
| Example 8B (ALPHASAN® RC 2000) | 42.20 +/- 0.25 |

As described previously, any of the substrates described herein may be used alone as a wound care device, including fabrics and alginates. Additionally, one or more of these substrates may be joined together in any possible combination to form a composite, multi-layered, wound care device. The layers may be joined together through various techniques such as ultrasonic welding, heat or pressure lamination, the use of adhesives, needle punching, hydraulic needling, sewing, or other fiber and/or fabric layer laminating or joining processes known to those skilled in the art. The layers may be joined together only at intermittent locations or the layers may be joined together completely.

The topical antimicrobial finish of the current invention may be applied to any one or more of the substrate layers comprising the composite wound care device. Additionally, an odor absorbing agent or layer (such as activated carbon) may be included on or within one or more layers of the composite wound care device. Furthermore, in some instances, the wound care device may have an adhesive layer so that the device may be held in place over the wound site. In such cases, a layer of removable film may be placed over the wound-facing side of the wound care device to protect the adhesive layer until ready for use. Alternatively, the wound care device may be held in place by wrapping long pieces of wound dressing, such as gauze, over and around the wound care device and securing the free end in place by any suitable means, such as tape, adhesive, pins, clips, or hooks.

Thus, the above description and examples show that a topical antimicrobial finish may be applied to a variety of substrates to achieve an antimicrobially effective, silver-containing wound care device having the desired characteristics of antimicrobial efficacy, functional release of silver, absence of a dark color, and fluid transport. As has been described herein, the present wound care device possesses a significant advantage over competitive products, in that it exhibits a white color uncharacteristic of silver-containing antimicrobial articles, in that the white color is sustainable over long periods (i.e., in production, transit, and storage), and in that it possesses unique fluid transport properties.

## Claims

1. A wound care device comprising:
a fabric having a wound contact surface and a wound fluid reservoir surface,
wherein said wound contact surface is comprised primarily of hydrophobic fiber and said wound fluid reservoir surface is comprised primarily of hydrophilic fiber, said hydrophobic and hydrophilic fibers being intermeshed together in a jersey knit construction, wherein the jersey knit is a circular or flat-knit fabric made with a plain stitch in which the loops intermesh in only one direction; and
wherein said wound care device transports wound fluid uni-directionally from said wound contact surface to said wound fluid reservoir surface upon exposure to a wound,
wherein at least said wound contact surface is coated with a composition comprising at least one silver ion-releasing compound, said compound is a silver ion exchange material, said silver ion exchange material being selected from the group consisting of silver zirconium phosphate, silver calcium phosphate, silver zeolite, and mixtures thereof.

2. The wound care device of Claim 1, wherein said hydrophobic fiber is polyester fiber.

3. The wound care device of Claim 1, wherein said hydrophilic fiber is polyamide fiber.

4. The wound care device of claim 1, wherein said knit fabric further comprises an elastomeric fiber.

5. The wound care device of Claim 2, wherein said composition further comprises a binding agent selected from the group consisting of polyurethane binders, acrylic binders, and mixtures thereof.

6. The wound care device of Claim 5, wherein said binding agent is a polyurethane-based material.

7. The wound care device of Claim 1, wherein said wound fluid reservoir surface is coated with a composition comprising at least one compound delivering silver ions.

## Patentansprüche

1. Wundpflegeartikel, umfassend:
ein Gewebe, das eine Wundkontaktfläche und eine Reservoirfläche für Wundflüssigkeit aufweist,
in dem die Wundkontaktfläche vorwiegend hydrophobe Fasern umfasst und die Reservoirfläche für Wundflüssigkeit vorwiegend hydrophile Fasern umfasst, wobei hydrophobe und hydrophile Fasern in einer Jersey-Strickkonstruktion miteinander vermascht sind, wobei der Jersey-Strick ein kreisförmiges oder ein flachgestricktes Gewebe ist, das aus rechten Maschen (plain stich) hergestellt ist, in dem die Schleifen nur in eine Richtung ineinander greifern; und
wobei der Wundpflegeartikel bei Kontakt mit einer Wunde die Wundflüssigkeit unidirektional von der Wundkontaktfläche auf die Reservoirfläche für Wundflüssigkeit transportiert,
wobei zumindest die Wundkontaktfläche mit einer Zusammensetzung beschichtet ist, die mindestens eine Silberionen freisetzende Verbindung umfasst, diese Verbindung ein Silberionenaustauschmaterial ist, wobei dieses Silberionenaustauschmaterial aus der Gruppe, bestehend aus Silber-Zirkoniumphosphat, Silber-Calciumphosphat, Silber-Zeolith und deren Mischungen ausgewählt ist.

2. Wundpflegeartikel nach Anspruch 1, in dem die hydrophobe Faser eine Polyesterfaser ist.

3. Wundpflegeartikel nach Anspruch 1, in dem die hydrophile Faser eine Polyamidfaser ist.

4. Wundpflegeartikel nach Anspruch 1, in dem das gestrickte Gewebe zusätzlich eine Elastomer-Faser umfasst.

5. Wundpflegeartikel nach Anspruch 2, in dem die Zusammensetzung ferner ein Bindemittel umfasst, das aus der Gruppe bestehend aus Polyurethan-Bindemitteln, Acrylbindemitteln und deren Mischungen ausgewählt ist.

6. Wundpflegeartikel nach Anspruch 5, in dem das Bindemittel ein Material auf Polyurethanbasis ist.

7. Wundpflegeartikel nach Anspruch 1, in dem die Reservoirfläche für Wundflüssigkeit mit einer Zusammensetzung beschichtet ist, die mindestens eine Silberionen-freisetzende Verbindung umfasst.

## Revendications

1. Dispositif de soins des plaies comprenant :
une étoffe ayant une surface de contact avec les plaies et une surface formant réservoir de fluide de plaie,
où ladite surface de contact avec les plaies comprend principalement une fibre hydrophobe et ladite surface formant réservoir de fluide de plaie comprend principalement une fibre hydrophile, lesdites fibres hydrophobe et hydrophile étant entremêlées entre elles dans une construction de tricot jersey, où le tricot jersey est une étoffe tricotée circulaire ou plate faite avec un point ordinaire où les boucles s'entremêlent dans une direction seulement ; et
où ledit dispositif de soins des plaies transporte du fluide de plaie de manière unidirectionnelle depuis ladite surface de contact avec les plaies jusqu'à ladite surface formant réservoir de fluide de plaie lors d'une exposition à une plaie,
où au moins ladite surface de contact avec les plaies est revêtue d'une composition comprenant au moins un composé libérant des ions argent, ledit composé est un matériau d'échange d'ions argent, ledit matériau d'échange d'ions argent étant choisi dans le groupe consistant en le phosphate d'argent et de zirconium, le phosphate d'argent et de calcium, une zéolite à l'argent et leurs mélanges.

2. Dispositif de soins des plaies selon la revendication 1, où ladite fibre hydrophobe est une fibre de polyester.

3. Dispositif de soins des plaies selon la revendication 1, où ladite fibre hydrophile est une fibre de polyamide.

4. Dispositif de soins des plaies selon la revendication 1, où ladite étoffe tricotée comprend en outre une fibre élastomère.

5. Dispositif de soins des plaies selon la revendication 2, où ladite composition comprend en outre un agent liant choisi dans le groupe consistant en les liants polyuréthanes, les liants acryliques et leurs mélanges.

6. Dispositif de soins des plaies selon la revendication 5, où ledit agent liant est un matériau à base de polyuréthane.

7. Dispositif de soins des plaies selon la revendication 1, où ladite surface formant réservoir de fluide de plaie est revêtue d'une composition comprenant au moins un composé délivrant des ions argent.
